# EUROPEAN PATENT APPLICATION

(11) **EP 4 568 463 A1**
(43) Date of publication of application: **11.06.2025**
(21) Application number: 22952705.6
(22) Date of filing: 20.09.2022
(51) Int. Cl.: H10K 85/60, H10K 50/30, C07C 209/68, C07C 211/61

(54) **ORGANIC ELECTROLUMINESCENT DEVICE, ORGANIC ELECTROLUMINESCENT APPARATUS AND PHOTOELECTRIC DEVICE**

(30) Priority: 26.07.2022 CN 202210885193
(71) Applicant: JILIN OLED OPTICAL AND ELECTRONIC MATERIALS CO., LTD., Changchun, Jilin 130000 (CN)
(72) Inventor: WANG, Kang, Changchun, Jilin 130000 (CN); WANG, Tie, Changchun, Jilin 130000 (CN); REN, Weihua, Changchun, Jilin 130000 (CN); MA, Xiaoyu, Changchun, Jilin 130000 (CN); ZHANG, Xue, Changchun, Jilin 130000 (CN); ZHANG, Ying, Changchun, Jilin 130000 (CN)
(74) Representative: Gille Hrabal Partnerschaftsgesellschaft mbB Patentanwälte
(86) International application number: PCT/CN2022/119865
(87) International publication number: WO 2024/021264

(57) **Abstract**

The present invention relates to an organic electroluminescent device, an organic electroluminescent apparatus and a photoelectric device, and belongs to the technical field of organic electroluminescent apparatuses. The organic electroluminescent device comprises a first electrode, a second electrode, which faces the first electrode, a light-emitting layer, which is located between the first electrode and the second electrode, and a hole transport region, which is located between the first electrode and the light-emitting layer, wherein the hole transport region comprises a hole injection layer, a first hole transport layer and a second hole transport layer. Provided in the present invention is an organic electroluminescent device, which is manufactured by a specific combination of a specific arylamine derivative contained in a hole transport region and a dual-host red-light material contained in a light-emitting layer. The manufactured organic electroluminescent device has a high turning-on voltage and also has a relatively low driving voltage, and retains the device advantages of high efficiency and long service life of a dual-host red-light device. By means of increasing the turning-on voltage of a red-light device, the phenomenon of the color displayed being reddish caused by crosstalk is alleviated.

## Description

### TECHNICAL FIELD

The present disclosure relates to the technical field of organic electroluminescent apparatuses, and in particular to an organic electroluminescent device, an organic electroluminescent apparatus, and a photoelectric device.

### BACKGROUND

Organic light-emitting diode (OLED) displays are active light-emitting display apparatuses. Currently, small and medium-sized OLED display screens have been widely applied to high-end smartphones produced by Huawei, Xiaomi, Samsung, and other companies. It is a general demand in the field of OLEDs that the optimal luminous efficiency of devices can be obtained under low operating voltage conditions. Reducing the driving voltage is an important way to reduce power consumption and improve the luminous efficiency and stability of the devices. Currently, there are many methods to achieve low-voltage driving of the devices and further improve the luminous efficiency of the devices, one of which is to use an exciplex-forming cohost material (i.e., a dual-host material). The cohost material is generally composed of a hole transport-type host material (P-type) and an electron transport-type host material (N-type). Rohm & Haas and other companies have launched a series of products, which have been applied to production lines, achieving the effects of low driving voltage, high efficiency, and long service life.

The turn-on voltage is defined as a voltage that needs to be applied when the luminance of a device reaches 1 nit. In the existing OLED display devices, the turn-on voltages of RGB three-color sub-pixels are inconsistent. Specifically, the turn-on voltage of the blue sub-pixel is greater than that of the green sub-pixel, which is greater than that of the red sub-pixel. In practical application, the blue sub-pixel has a relatively large efficiency difference under different driving voltage conditions. The blue sub-pixel has low luminous efficiency when driving at a low-voltage. However, the red sub-pixel and the green sub-pixel have relatively low driving voltages, and thus can normally emit light when driving at the low-voltage. Therefore, the luminous efficiency of the red sub-pixel is higher than that of the green sub-pixel and the blue sub-pixel under the condition of low gray scale, so that a phenomenon of the screen color reddening occurs, and thus a crosstalk phenomenon occurs.

The phenomenon of the color reddening caused by the crosstalk can be improved by increasing the turn-on voltage of a red-light device. Although a single-host red-light device has a higher turn-on voltage than that of a dual-host red-light device, it has disadvantages of low luminous efficiency and short service life, while the dual-host red-light device generally has disadvantage of the increase in the driving voltage as the increase in the turn-on voltage.

### SUMMARY

The object of the present disclosure is to provide an organic electroluminescent device, an organic electroluminescent apparatus, and a photoelectric device, so as to solve the problems described above.

In order to achieve the object described above, the present disclosure provides the following technical solutions:
Provided is an organic electroluminescent device, comprising a first electrode, a second electrode facing the first electrode, a light emitting layer between the first electrode and the second electrode, and a hole transport region between the first electrode and the light emitting layer, wherein the hole transport region comprises a hole injection layer, a first hole transport layer, and a second hole transport layer;
wherein the material of the second hole transport layer has a general structural formula shown as formula I: 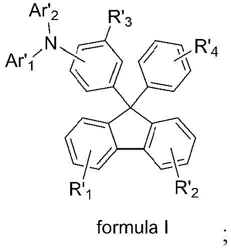 wherein
   Ar'₁ and Ar'₂ are each independently substituted or unsubstituted C₆-C₂₄ aryl, or substituted or unsubstituted 3- to 24-membered heteroaryl;
   R'₁-R'₃ are each independently hydrogen, deuterium, substituted or unsubstituted C₁-C₆ alkyl, substituted or unsubstituted C₆-C₁₈ aryl, substituted or unsubstituted 3- to 18-membered heteroaryl, substituted or unsubstituted C₃-C₁₂ cycloalkyl, or substituted or unsubstituted C₁-C₆ alkoxy;
   R'₄ is hydrogen, methyl, ethyl, phenyl, or biphenyl;
   R'₁-R'₄ are each independently bonded or fused to respective phenyl rings;
   the light emitting layer comprises a P-type host material and an N-type host material;
   wherein the P-type host material has a general structural formula shown as formula II: 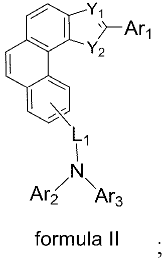 wherein
      Y₁ and Y₂ are in accordance with one of the following conditions:
      Y₁ is N, and Y₂ is O;
      Y₁ is N, and Y₂ is S;
      Y₁ is O, and Y₂ is N; and
      Y₁ is S, and Y₂ is N;
      L₁ is a linking bond, substituted or unsubstituted C₆-C₁₈ arylene, or substituted or unsubstituted 3- to 18-membered heteroarylene;
      Ar₁-Ar₃ are each independently substituted or unsubstituted C₆-C₂₄ aryl, or substituted or unsubstituted 3- to 24-membered heteroaryl;
      Ar₁-Ar₃ are bonded or fused to respective phenyl rings; and
      the N-type host material has a general structural formula shown as formula III: 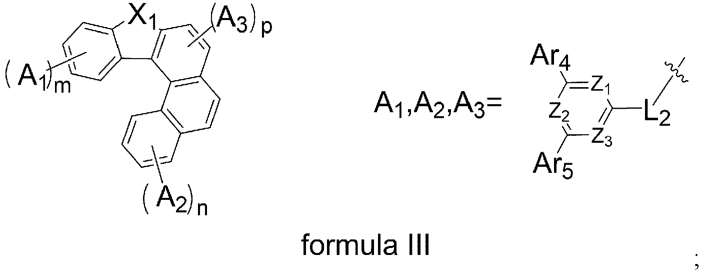 wherein
         X₁ is selected from O or S;
         one of m, n, and p is 1, and the rest are 0;
         Z₁-Z₃ are each independently N or C, and at least one of Z₁-Z₃ is N;
         Ar₄-Ar₅ are each independently substituted or unsubstituted C₆-C₂₄ aryl, or substituted or unsubstituted 3- to 24-membered heteroaryl; and
         L₂ is a linking bond, substituted or unsubstituted C₆-C₁₈ arylene, or substituted or unsubstituted 3- to 18-membered heteroarylene.

In a further embodiment of the present disclosure, the material of the second hole transport layer is selected from one of the following formulae I-1 to I-3: 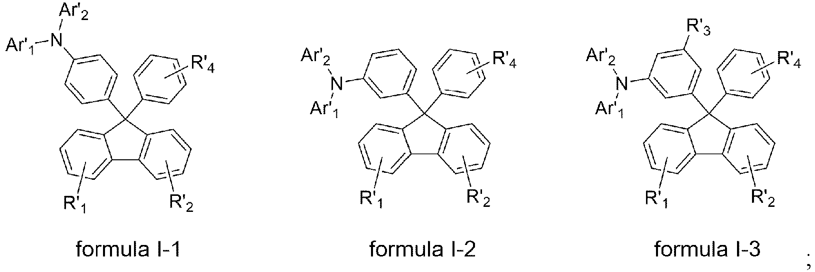 wherein
R'₁-R'₂ are each independently hydrogen, deuterium, methyl, ethyl, isopropyl, n-propyl, tert-butyl, phenyl, naphthyl, biphenyl, pyridinyl, methoxy, phenanthryl, dibenzofuranyl, or dimethylfluorenyl;
R'₃ is deuterium, methyl, ethyl, n-propyl, isopropyl, tert-butyl, phenyl, naphthyl, biphenyl, pyridinyl, methoxy, phenanthryl, dimethylfluorenyl, or furanyl;
Ar'₁ and Ar'₂ are linked to N at any substitutable position;
Ar'₁ and Ar'₂ are each independently one of the following groups or a combination thereof:

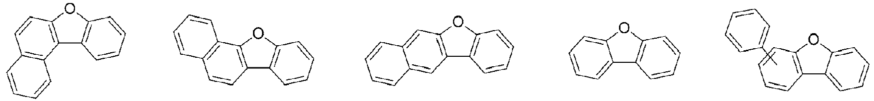

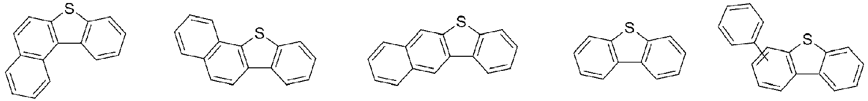

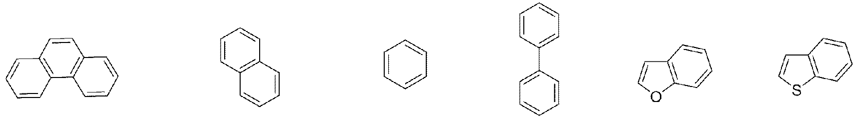

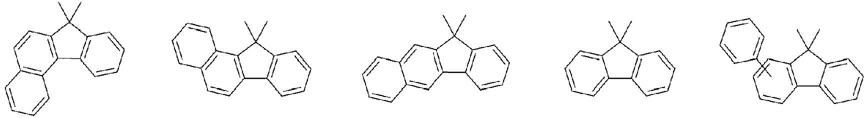

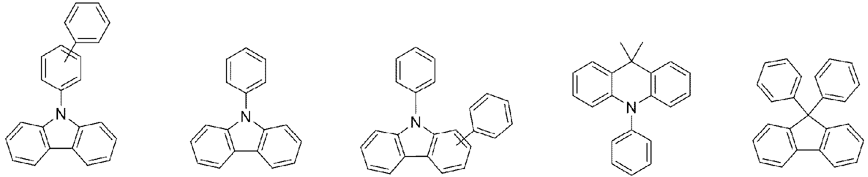

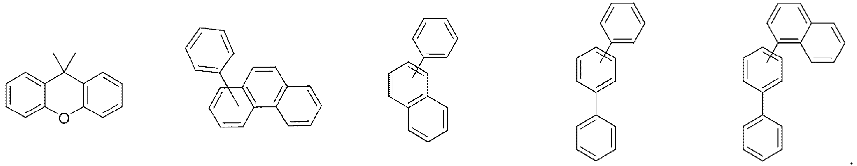

In a still further embodiment of the present disclosure, the material of the second hole transport layer is selected from one of the following HT-1 to HT-56:

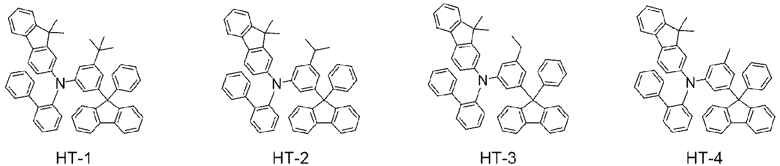

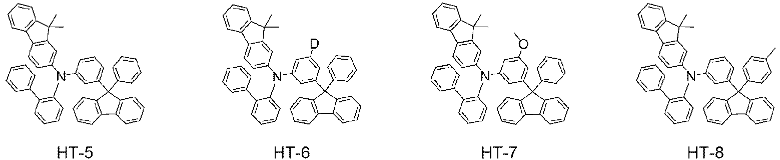

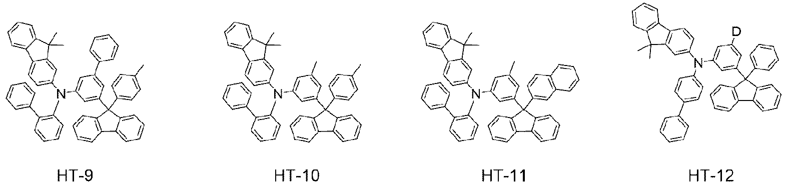

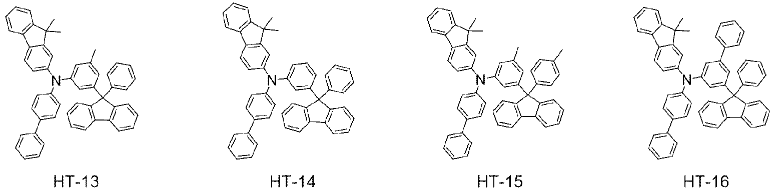

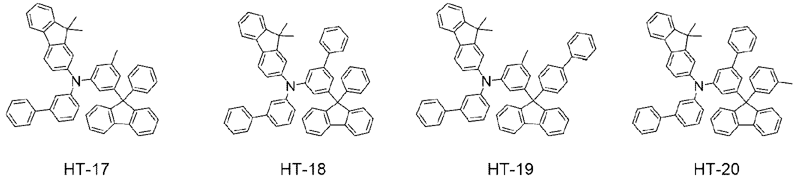

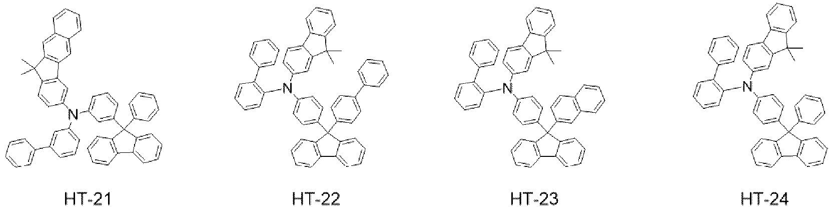

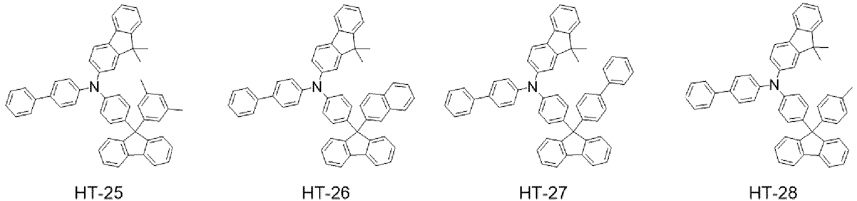

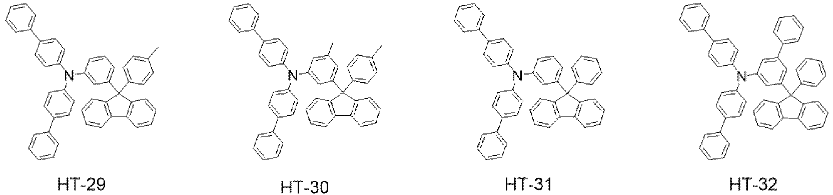

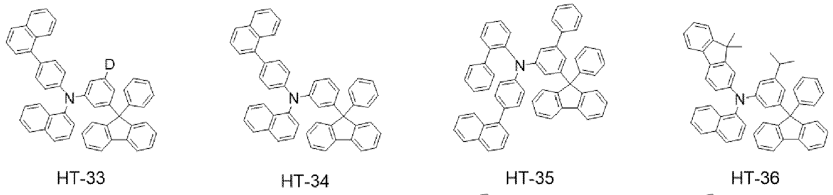

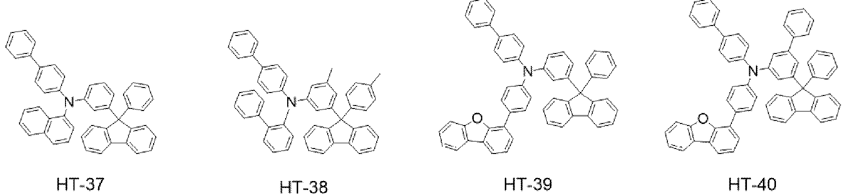

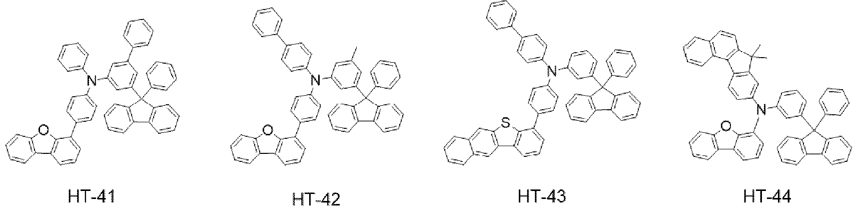

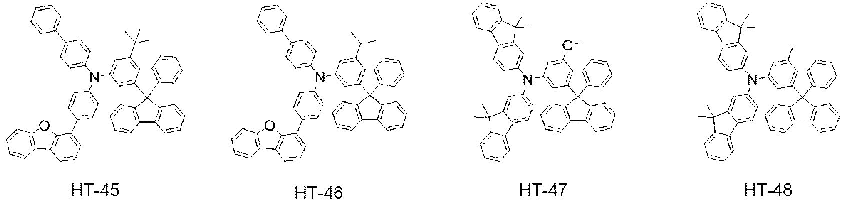

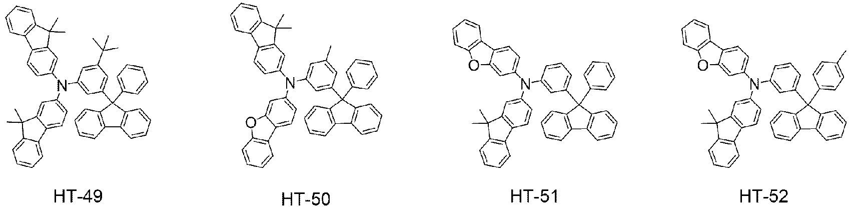

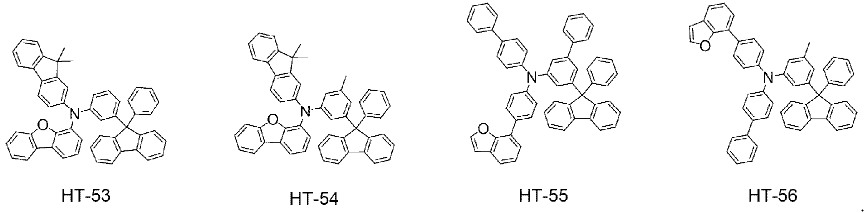

In a yet still further embodiment of the present disclosure, the P-type host material is selected from one of the following formulae II-a to II-d: 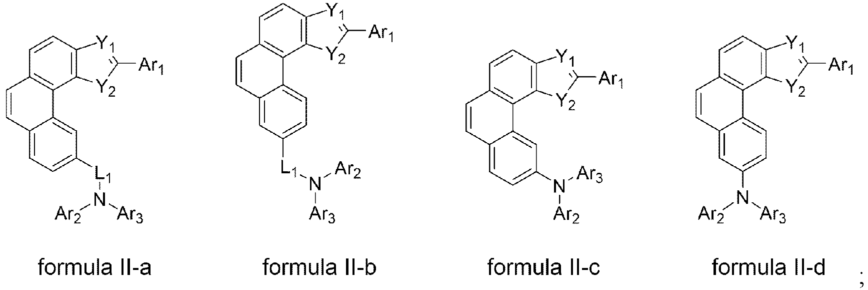 wherein
L₁ is a linking bond, phenyl, naphthyl, biphenyl, terphenyl, pyridinyl, phenanthryl, dibenzofuranyl, dibenzothienyl, or dimethylfluorenyl;
Ar₁-Ar₃ are each independently selected from phenyl, methylphenyl, ethylphenyl, isopropylphenyl, tert-butylphenyl, naphthyl, biphenyl, terphenyl, pyridinyl, phenanthryl, dibenzofuranyl, dibenzothienyl, dimethylfluorenyl, or carbazolyl.

In a yet still further embodiment of the present disclosure, the P-type host material is selected from one of the following formulae RH1-1 to RH1-85:

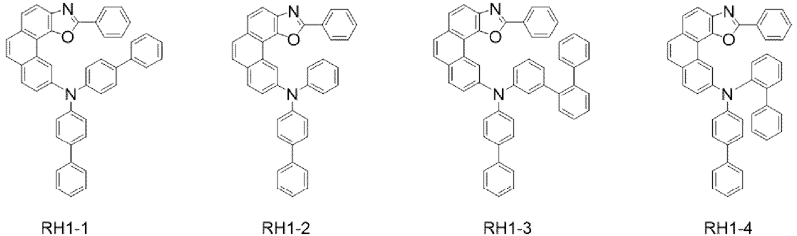

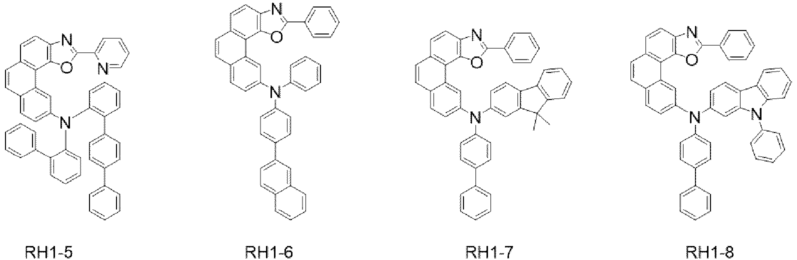

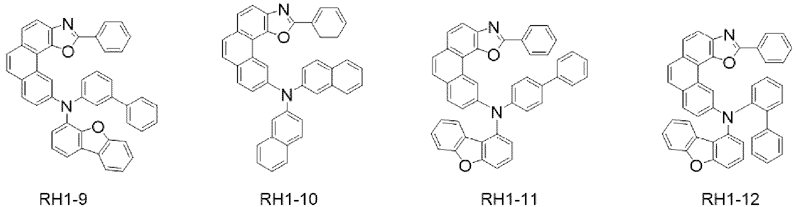

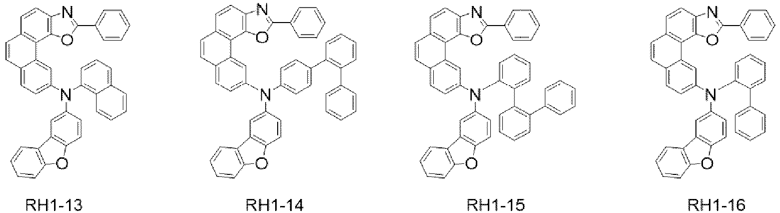

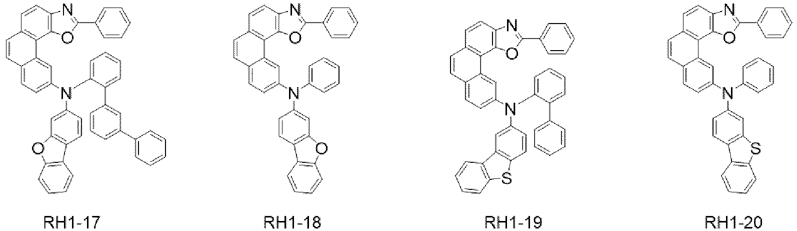

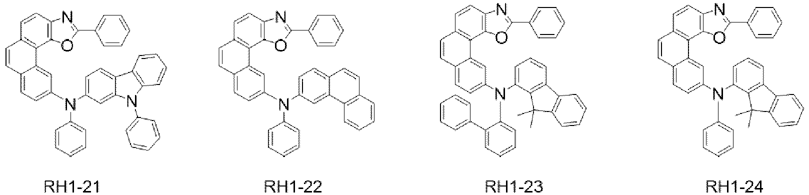

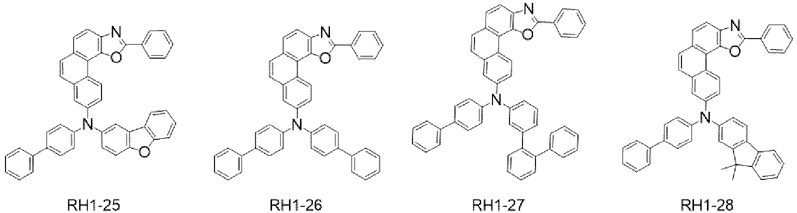

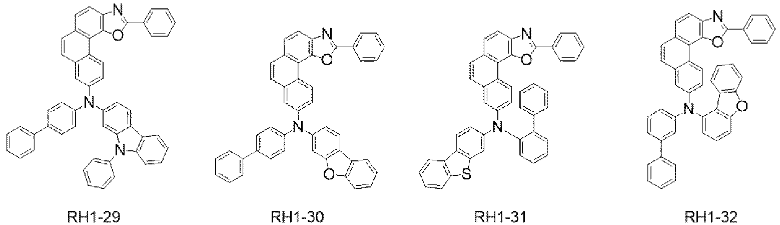

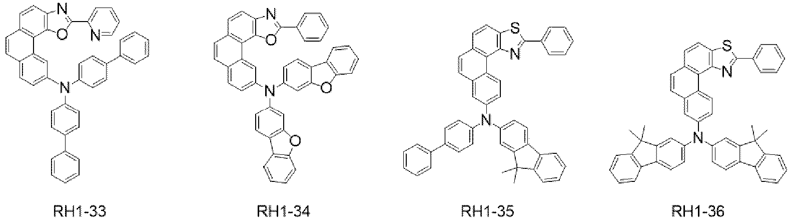

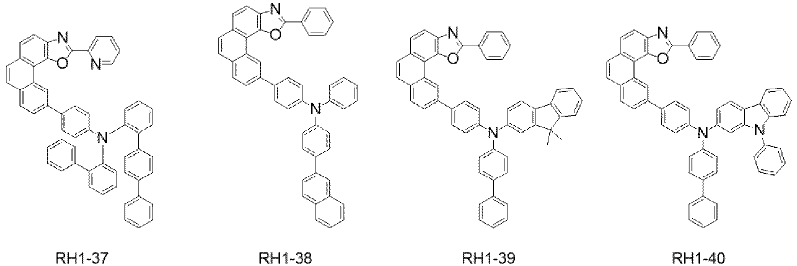

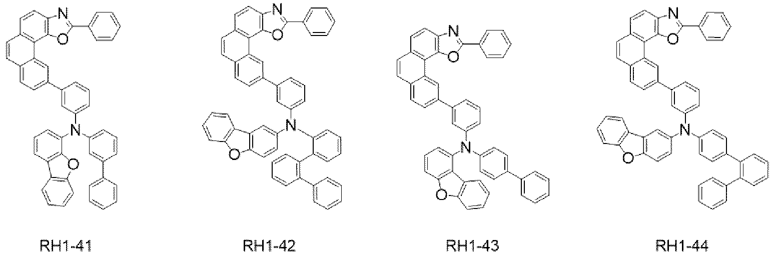

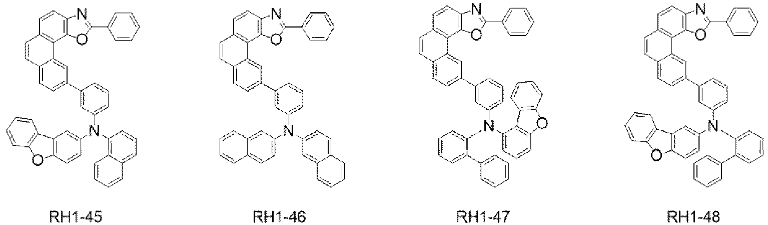

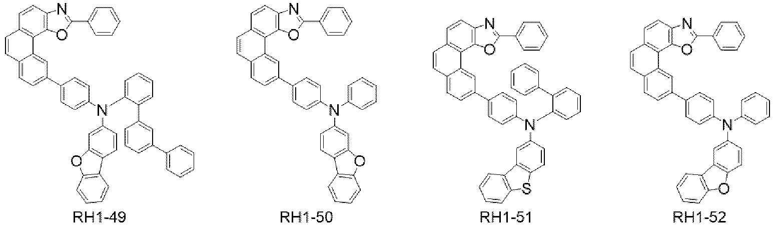

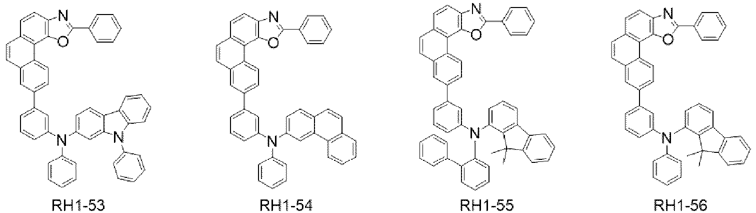

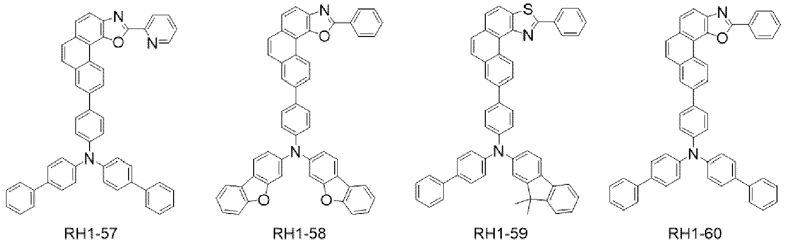

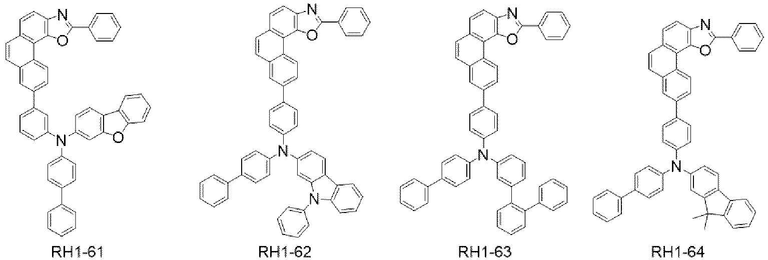

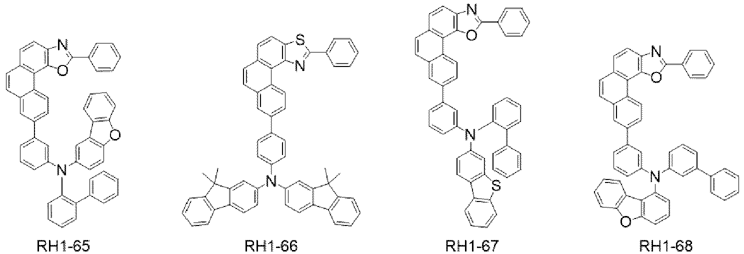

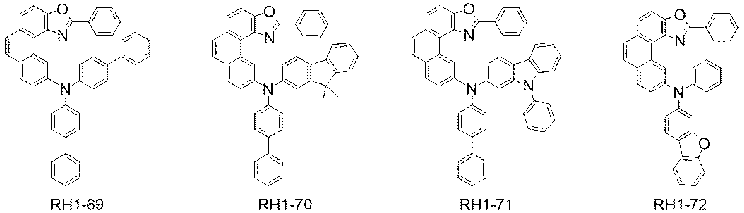

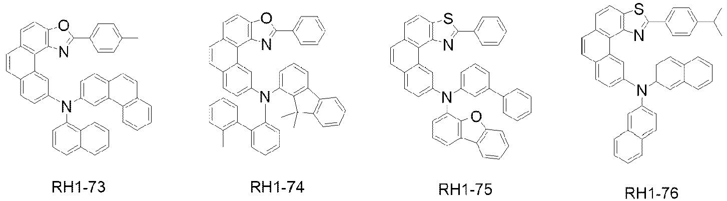

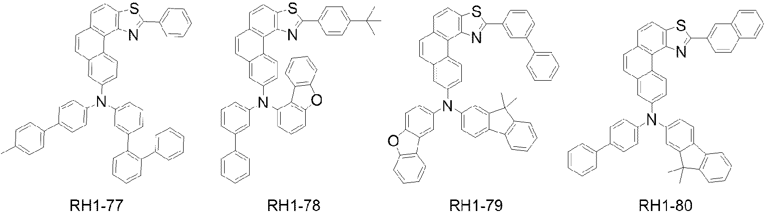

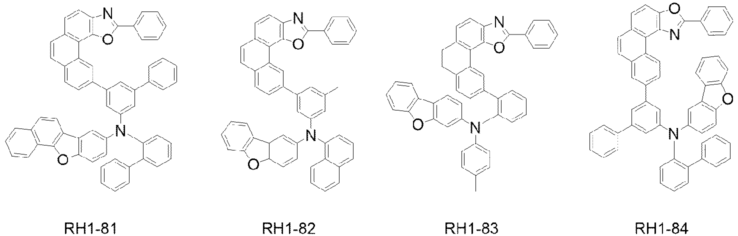

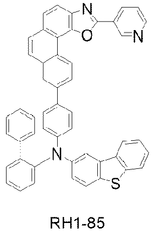

In a yet still further embodiment of the present disclosure, the N-type host material is selected from one of the following formulae III-a to III-c: 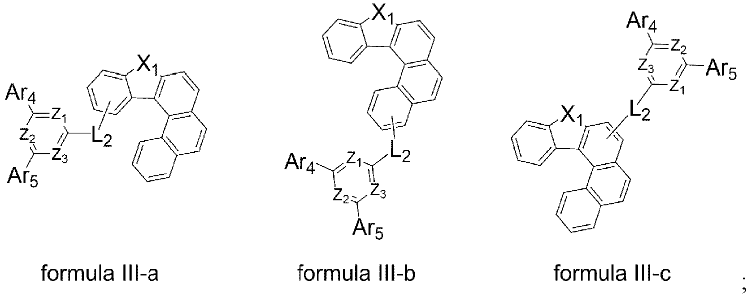 wherein
L₂ is a linking bond, phenyl, naphthyl, biphenyl, pyridinyl, phenanthryl, dibenzofuranyl, dibenzothienyl, or dimethylfluorenyl; and
Ar₄-Ar₅ are each independently selected from phenyl, methylphenyl, ethylphenyl, isopropylphenyl, tert-butylphenyl, naphthyl, biphenyl, terphenyl, pyridinyl, phenanthryl, dibenzofuranyl, dibenzothienyl, dimethylfluorenyl, or carbazolyl.

In a yet still further embodiment of the present disclosure, the N-type host material is selected from one of the following RH2-1 to RH2-96:

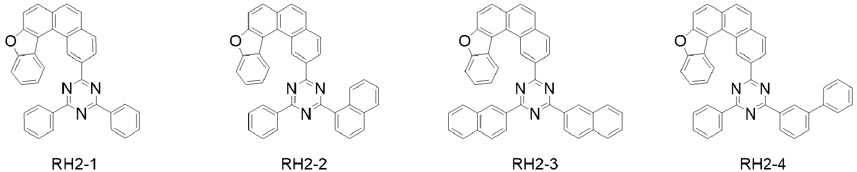

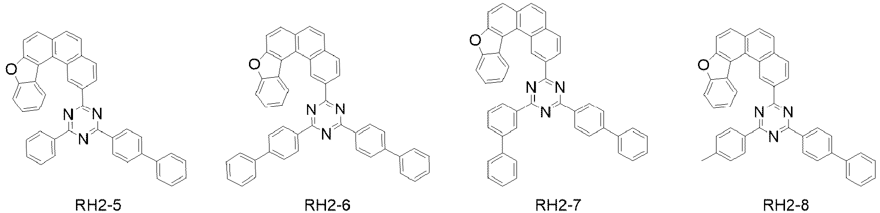

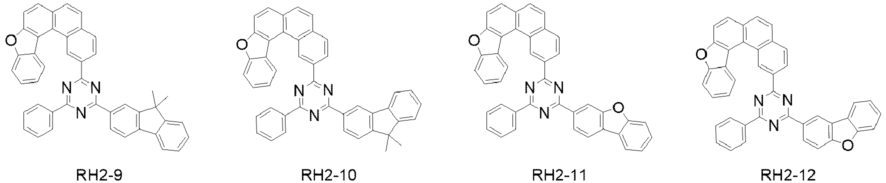

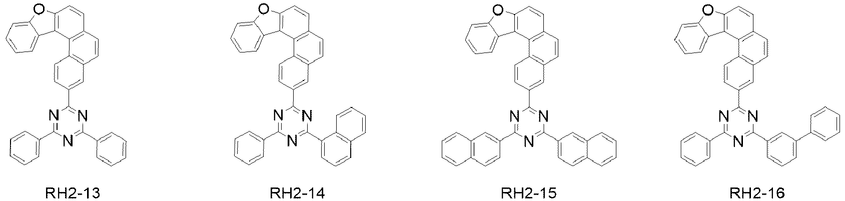

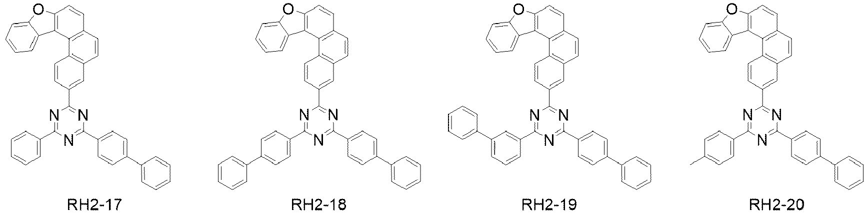

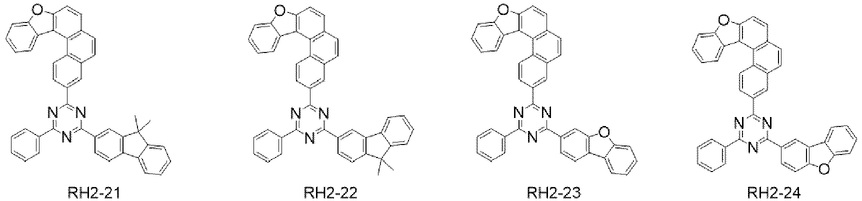

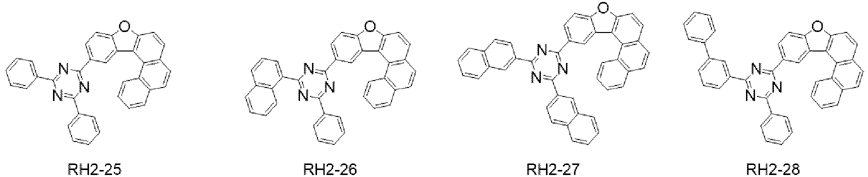

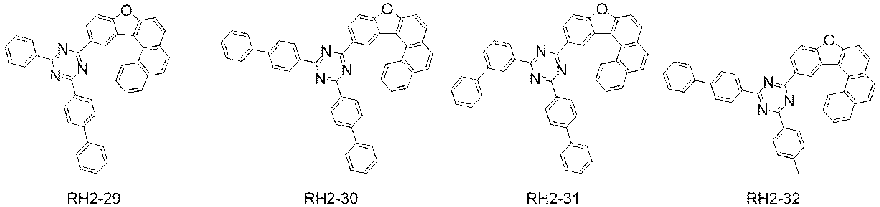

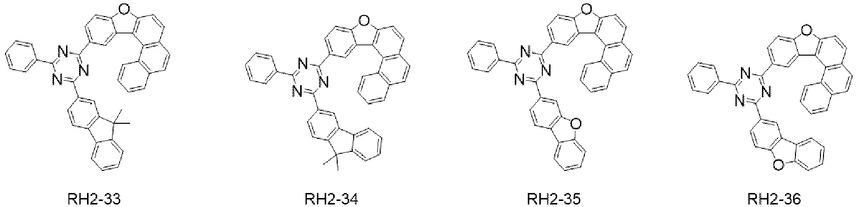

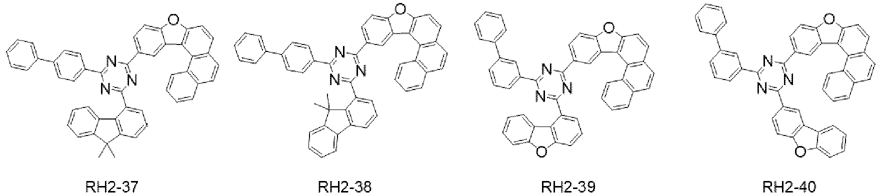

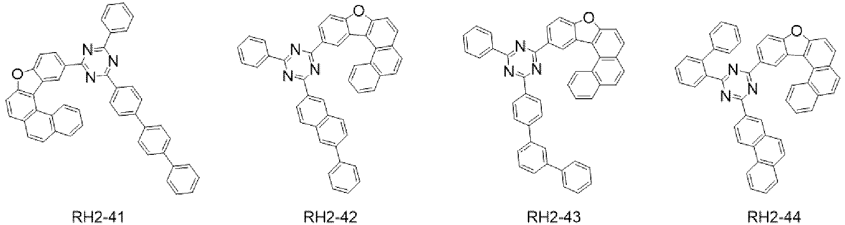

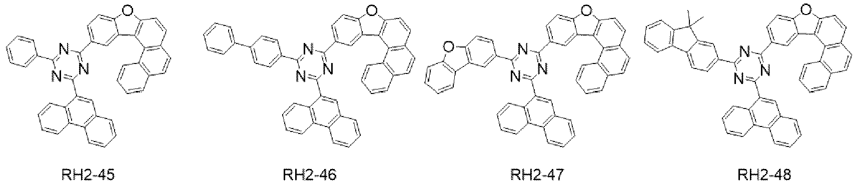

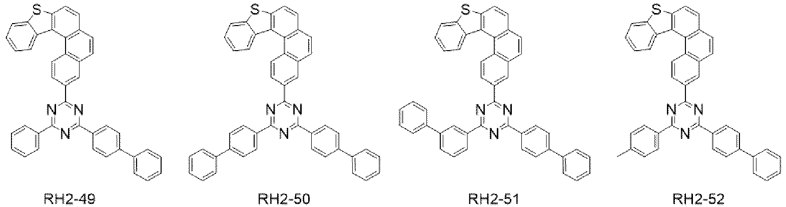

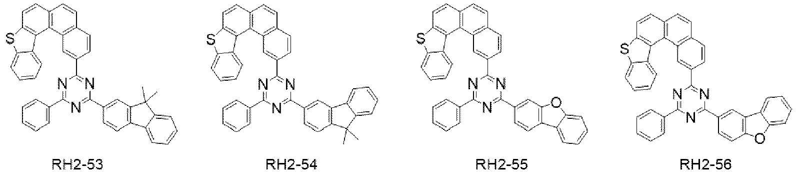

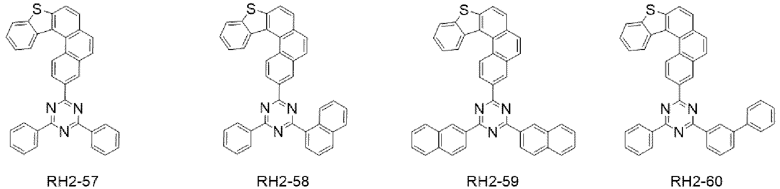

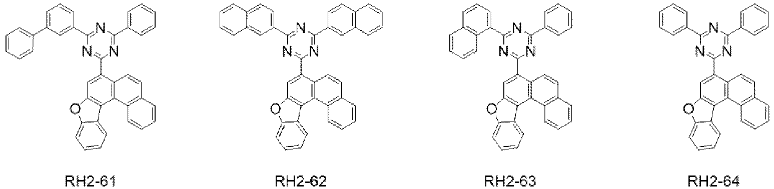

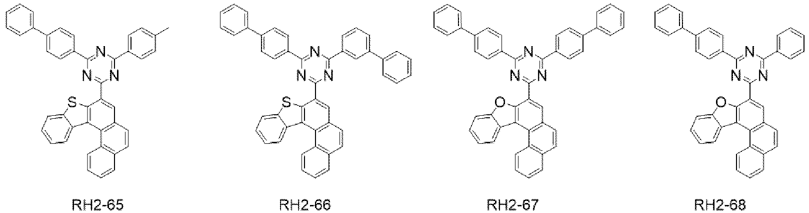

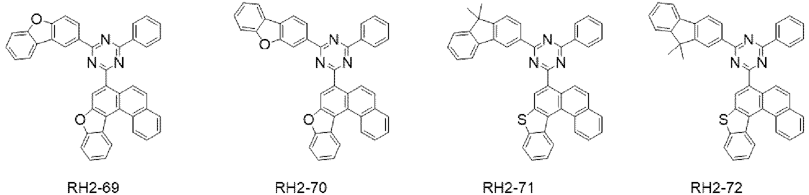

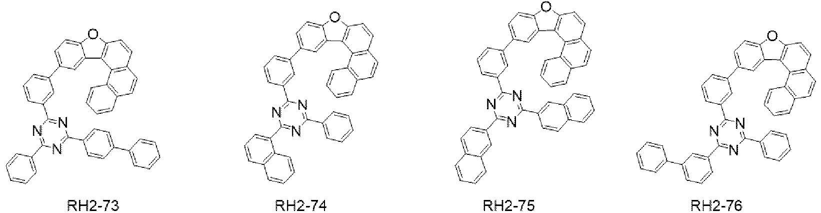

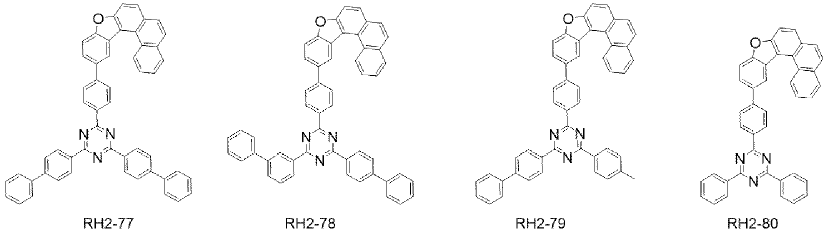

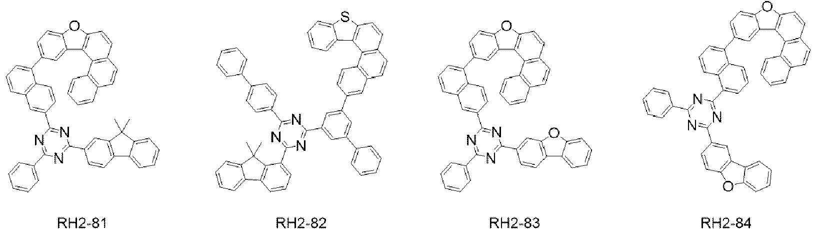

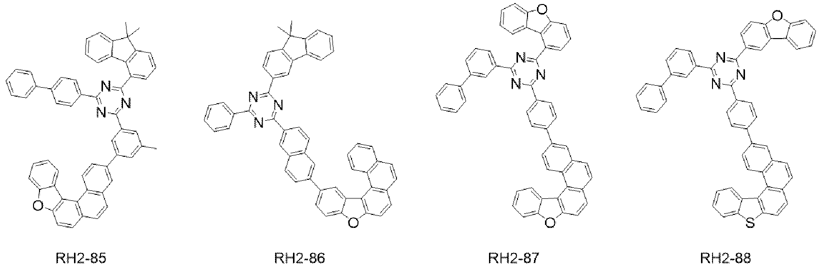

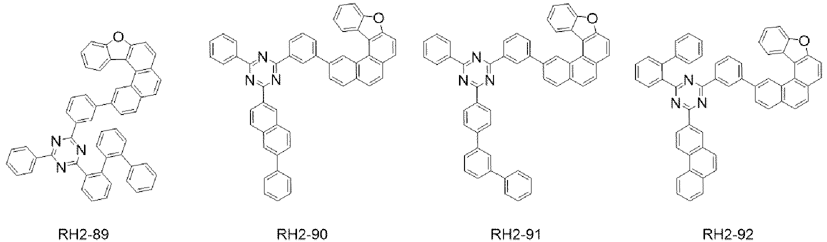

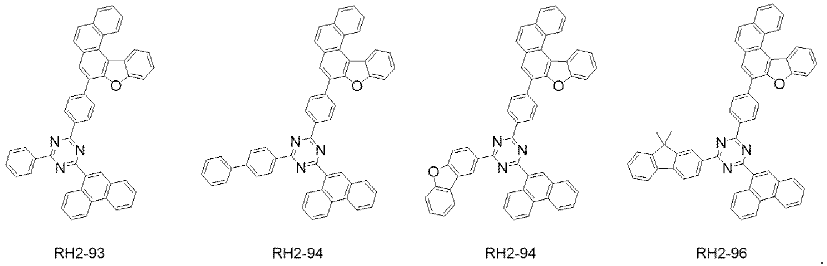

Compared with the prior art, the present disclosure has the beneficial effects that: provided is an organic electroluminescent device prepared by a specific combination of a specific arylamine derivative contained in a hole transport region and a dual-host red-light material contained in a light emitting layer; the prepared organic electroluminescent device has a high turn-on voltage and also has a relatively low driving voltage, and the device advantages of high efficiency and long service life of a dual-host red-light device are retained; and a phenomenon of the color reddening caused by crosstalk is alleviated by improving the turn-on voltage of the red-light device.

In this specification, the term "substituted or unsubstituted" means a group can be substituted with one, two, or more substituents selected from: deuterium; a halogen group; cyano; a silyl group; a boron group; C₁-C₆ alkyl; C₃-C₁₀ cycloalkyl; C₆-C₁₈ aryl; and C₃-C₃₀ heterocyclyl, or substituted with a substituent formed by linking two or more of the substituents shown above, or means a group is not substituted.

### BRIEF DESCRIPTION OF THE DRAWING

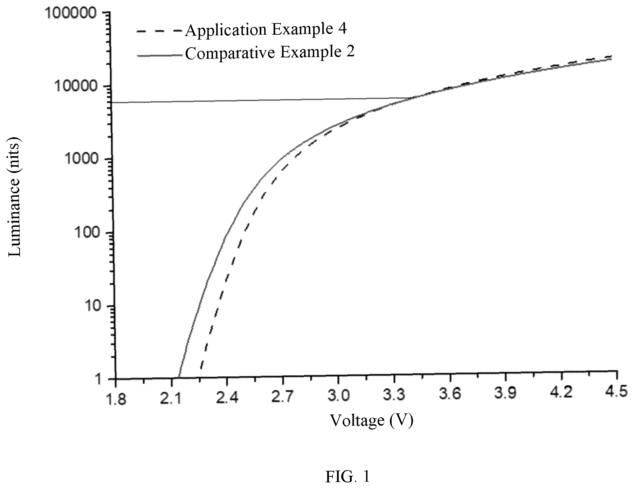

FIG. 1 is a graph showing the turn-on voltages of the organic electroluminescent devices prepared in Application Example 4 and Comparative Example 2.

### DETAILED DESCRIPTION

Provided is an organic electroluminescent device, comprising a first electrode, a second electrode facing the first electrode, a light emitting layer between the first electrode and the second electrode, and a hole transport region between the first electrode and the light emitting layer, wherein the hole transport region comprises a hole injection layer, a first hole transport layer, and a second hole transport layer;
wherein the material of the second hole transport layer has a general structural formula shown as formula I: 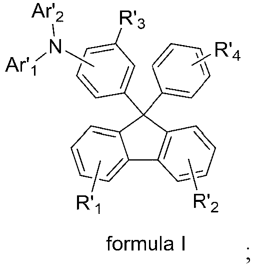 specifically, the material of the second hole transport layer is selected from one of the following HT-1 to HT-56: 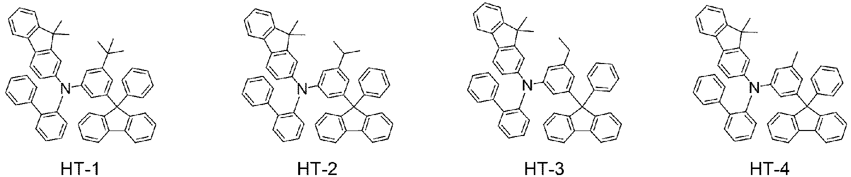 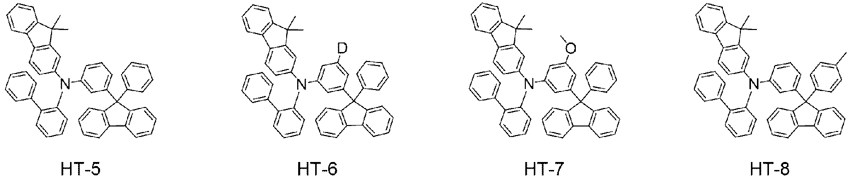 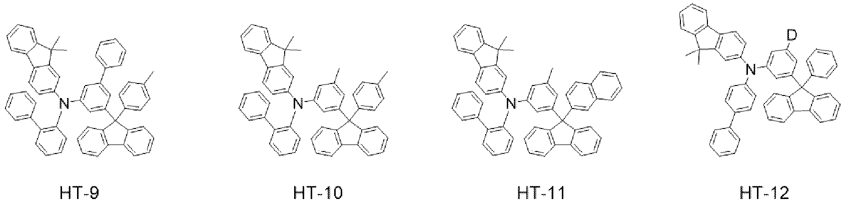 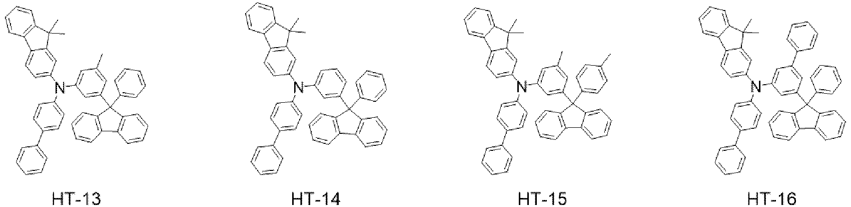 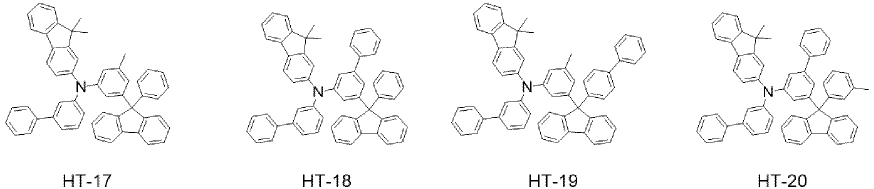 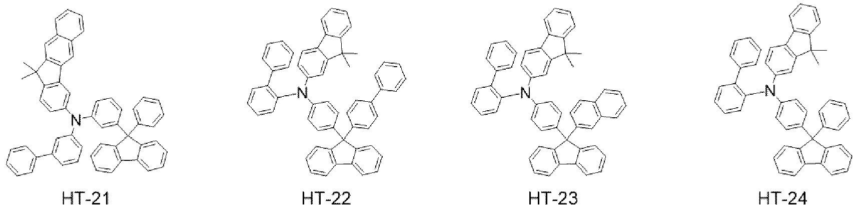 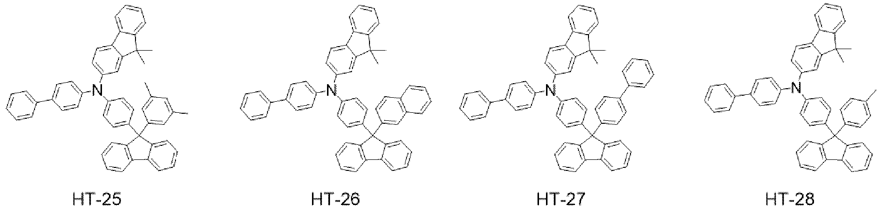 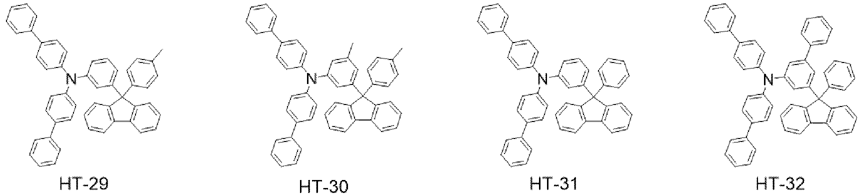 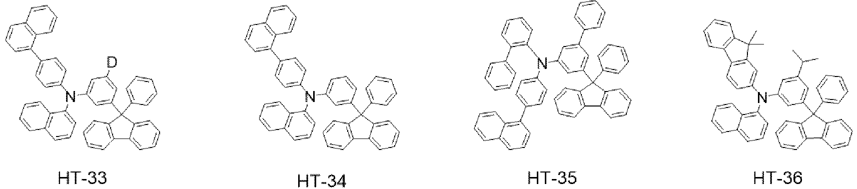 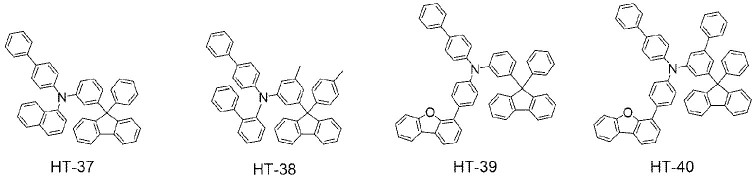 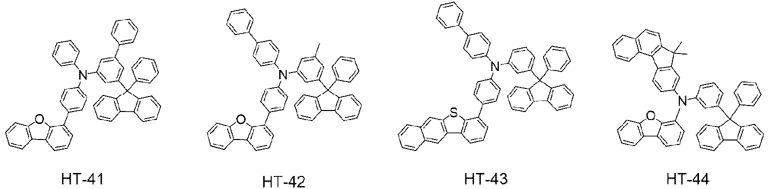 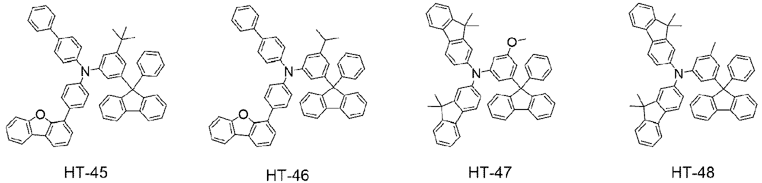 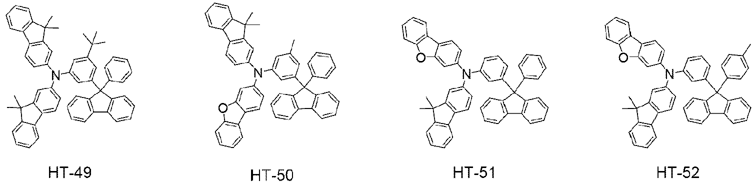 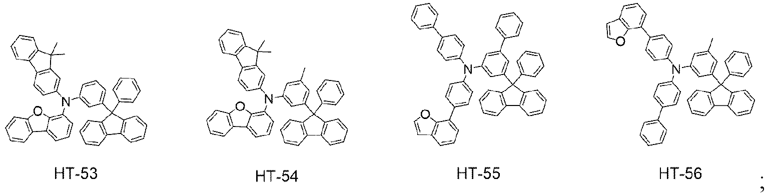 the light emitting layer comprises a P-type host material and an N-type host material; wherein the P-type host material has a general structural formula shown as formula II: 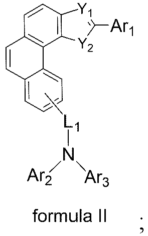 specifically, the P-type host material is selected from one of the following formulae RH1-1 to RH1-85: 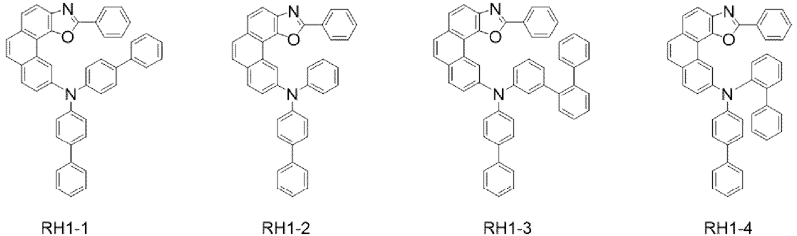 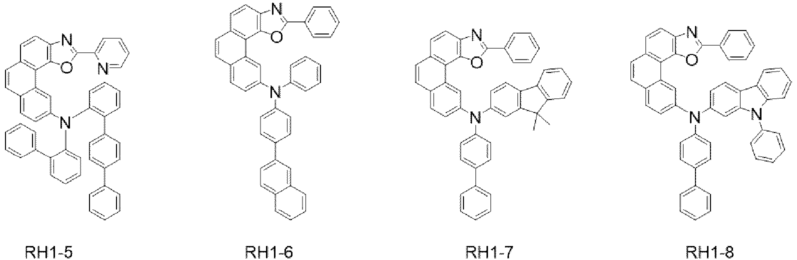 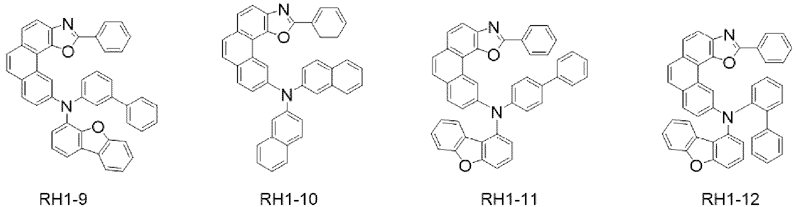 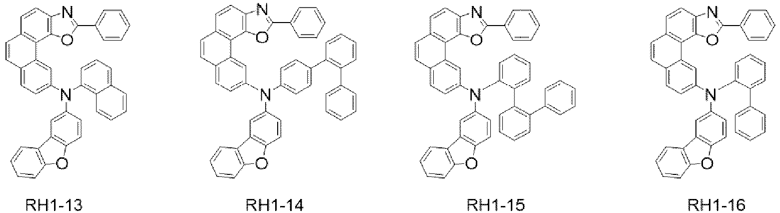 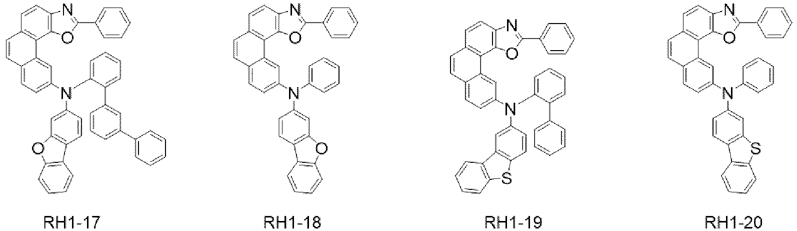 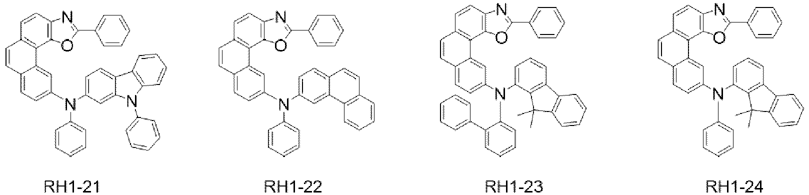 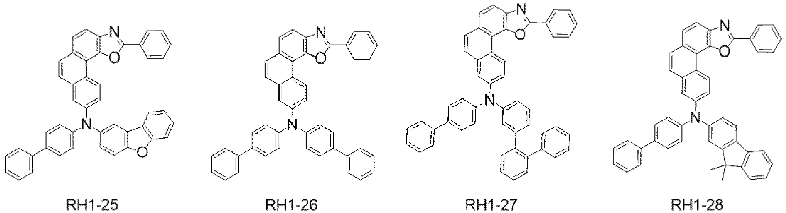 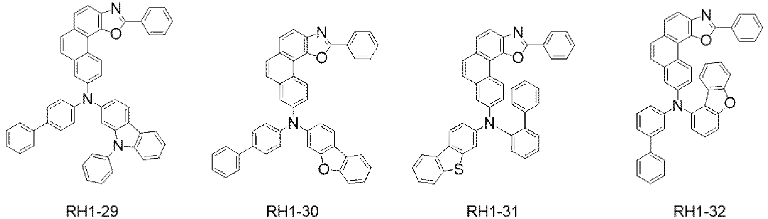 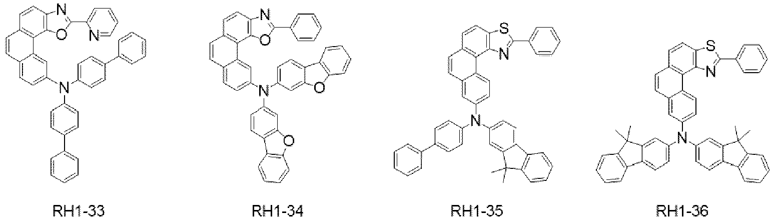 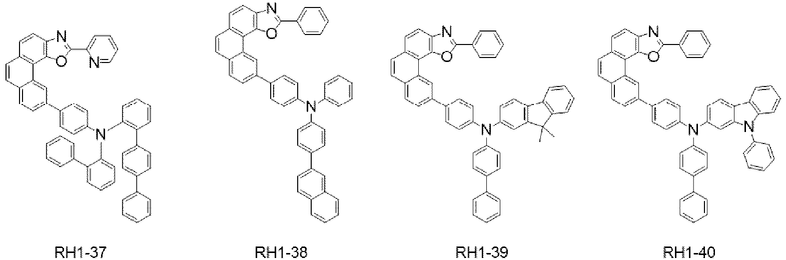 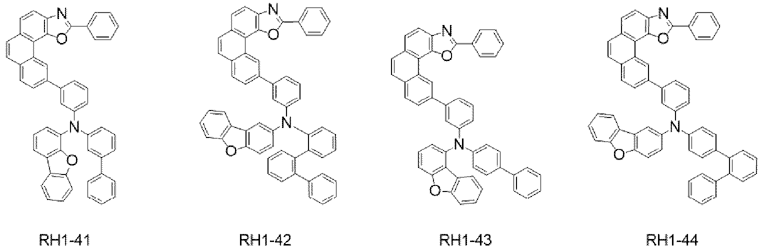 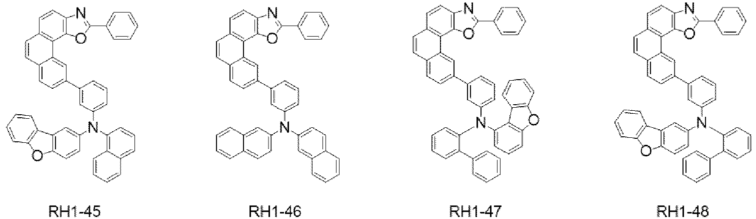 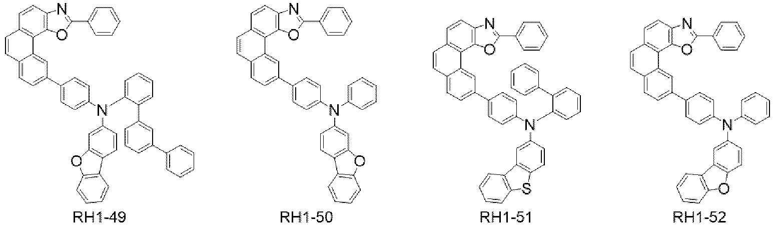 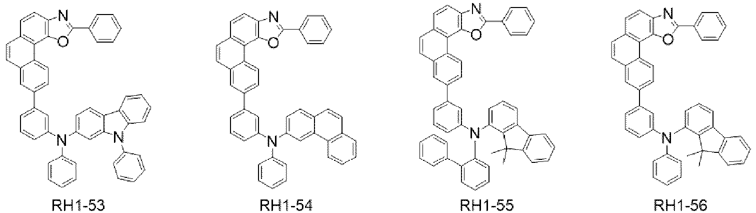 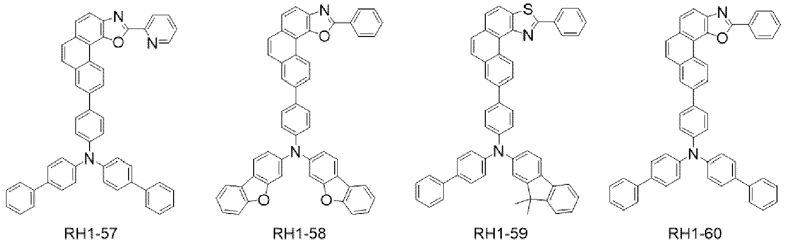 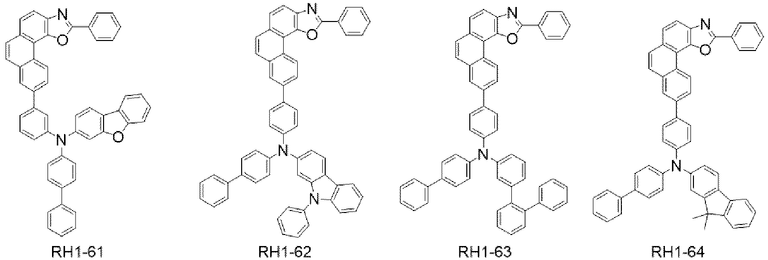 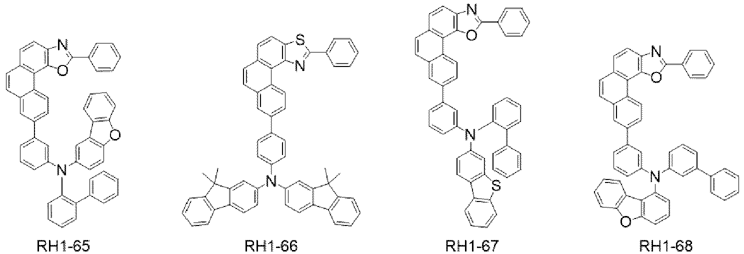 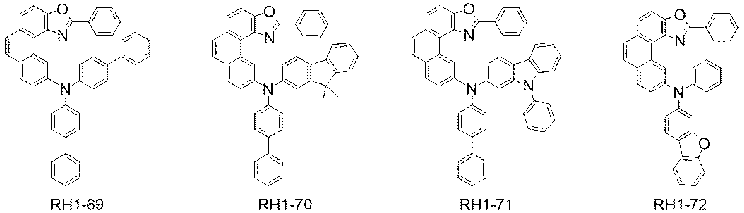 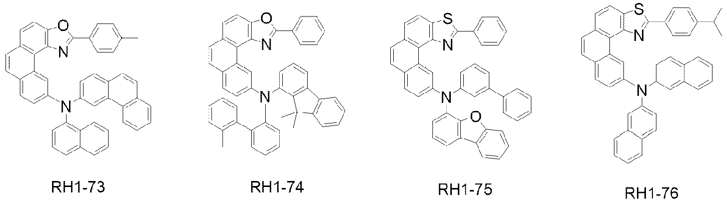 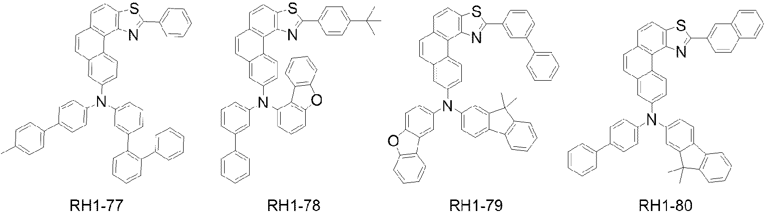 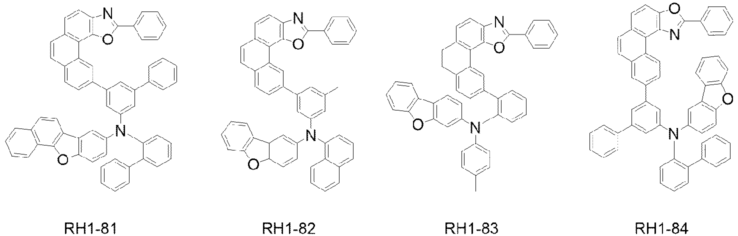 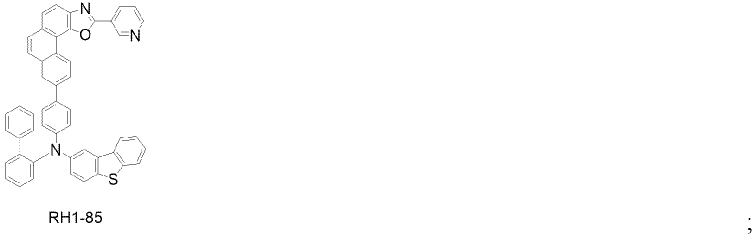 the N-type host material has a general structural formula shown as formula III, 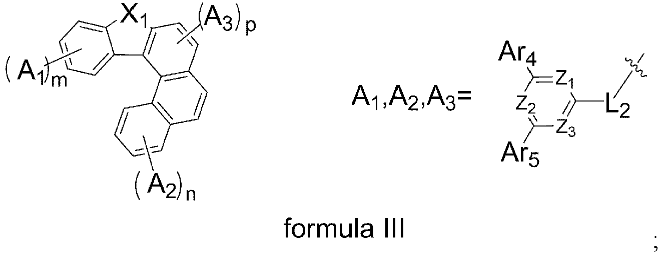 specifically, the N-type host material is selected from one of the following RH2-1 to RH2-96:

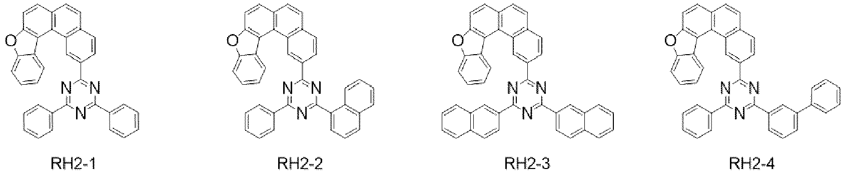

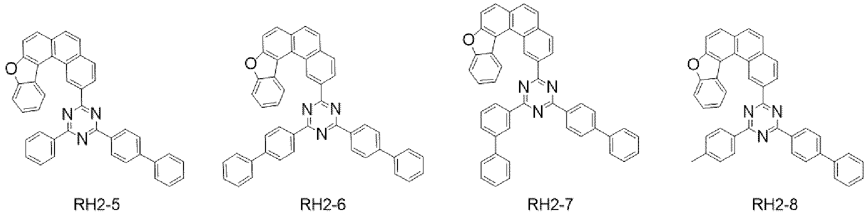

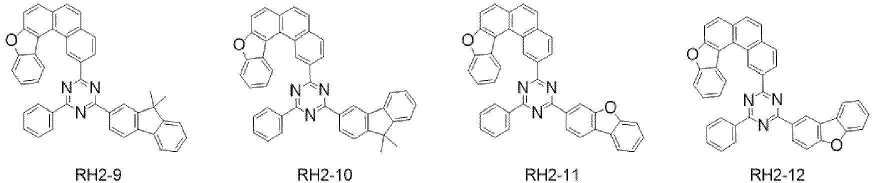

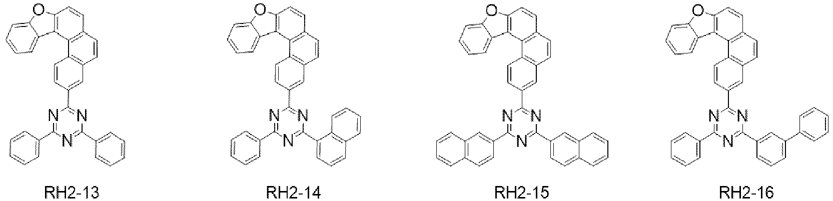

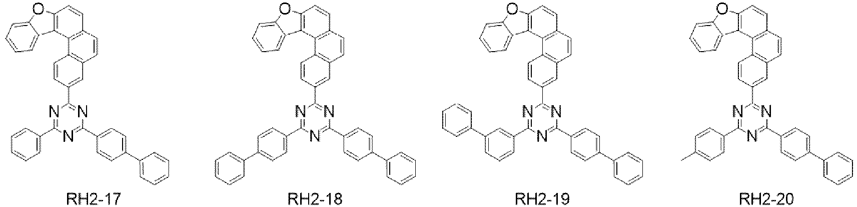

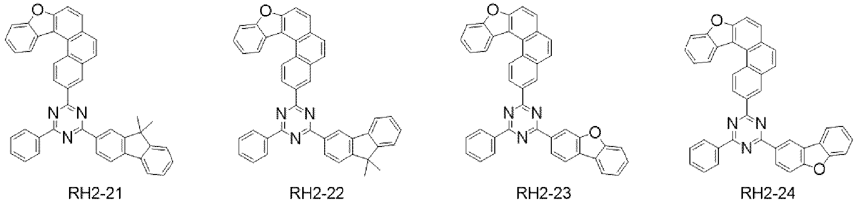

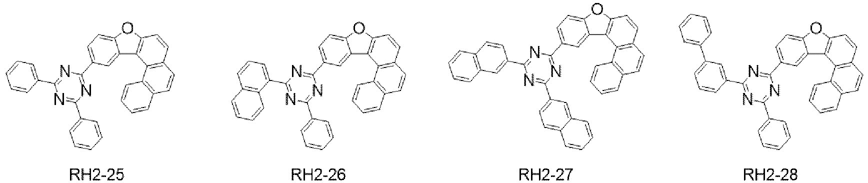

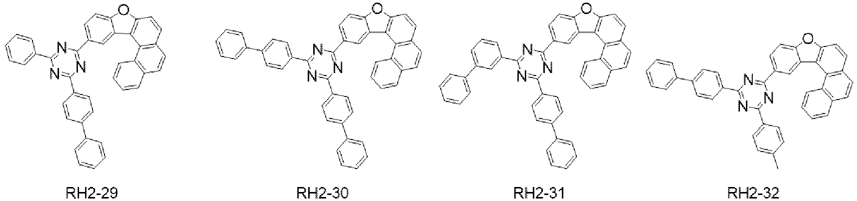

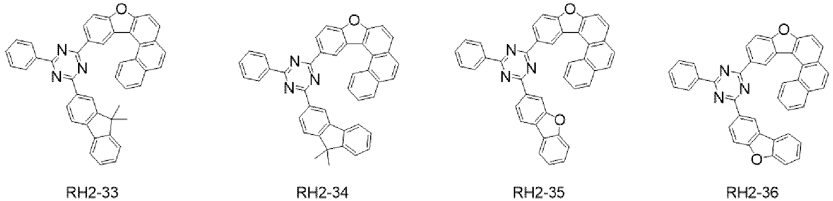

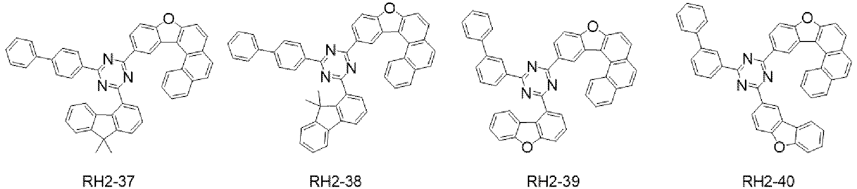

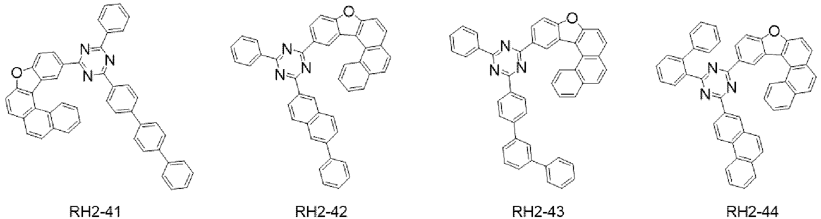

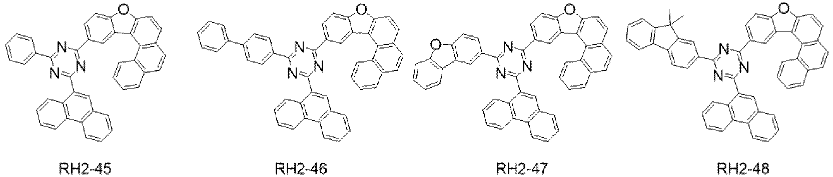

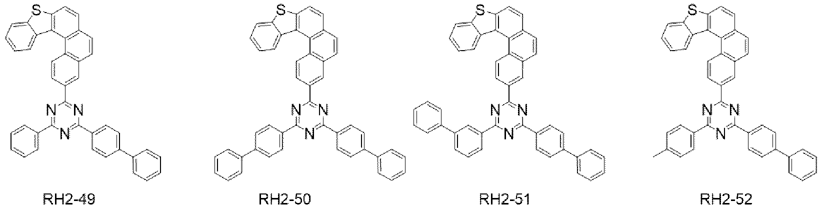

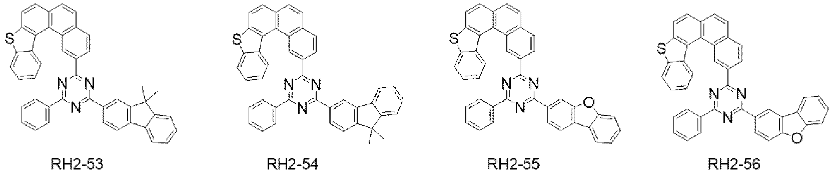

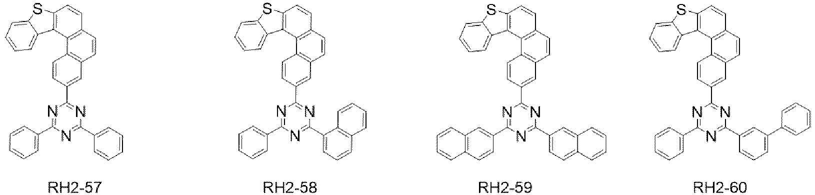

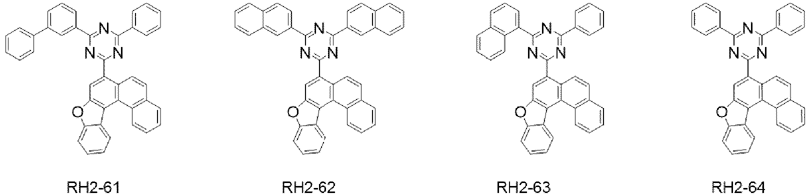

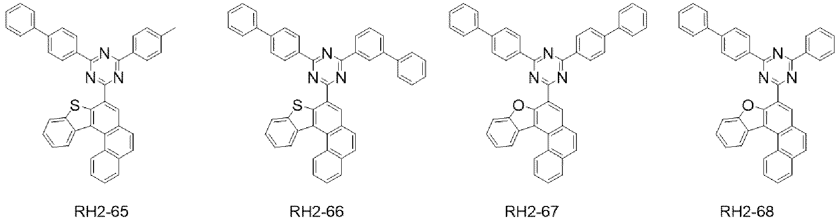

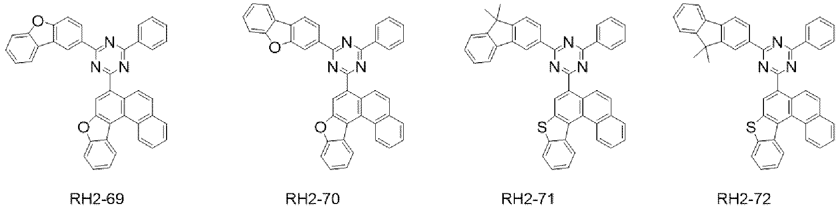

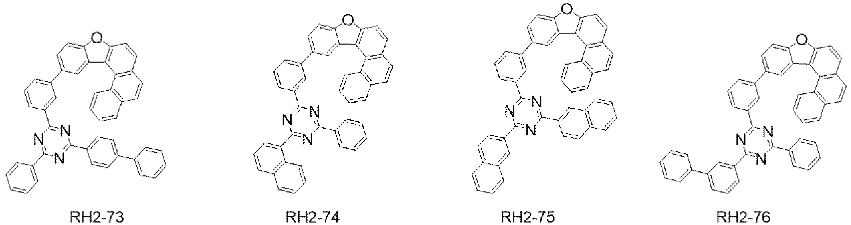

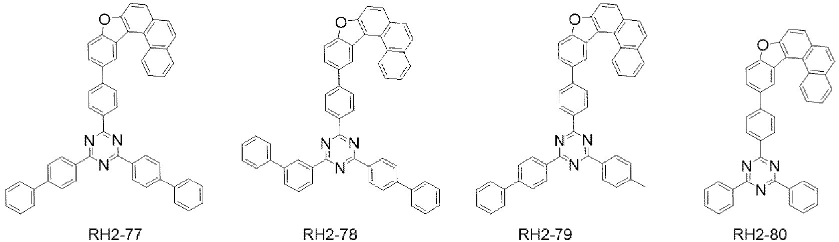

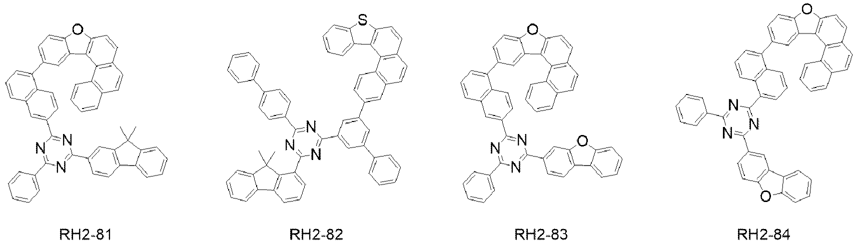

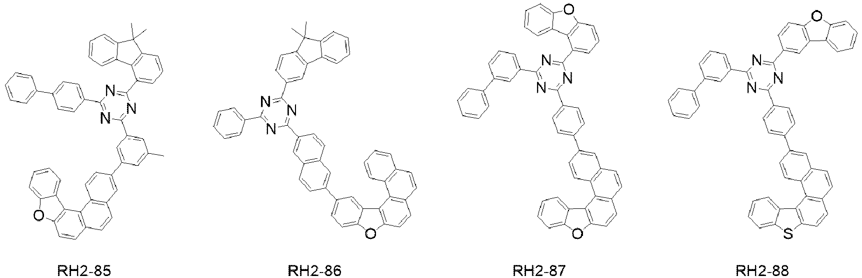

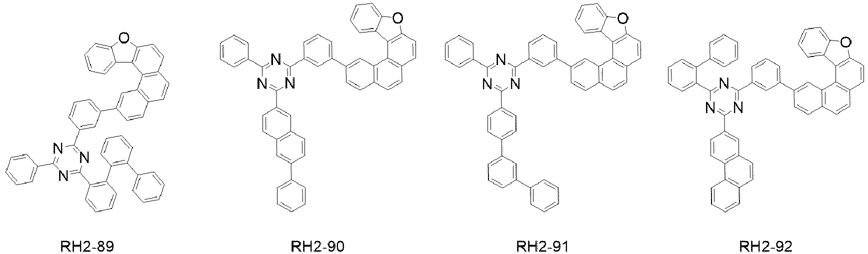

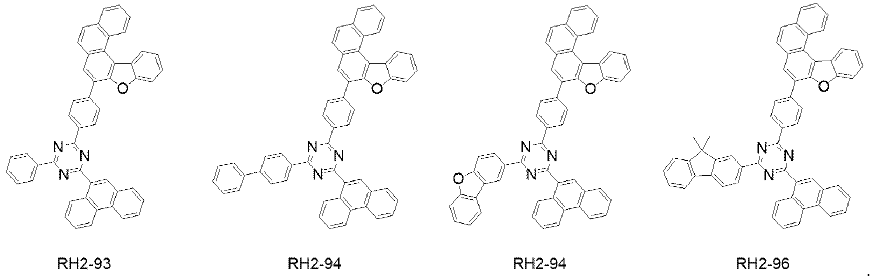

The synthetic route of the second hole transport layer in the organic electroluminescent device described above is as follows:

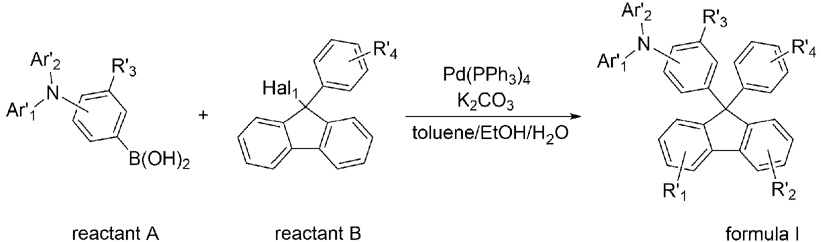

Under N₂ atmosphere, reactant A (1.0 eq), reactant B (1.0-1.3 eq), tetrakis(triphenylphosphine)palladium(0) (0.01-0.05 eq), and potassium carbonate (2.0-2.5 eq) are separately added to a mixed solvent of toluene, ethanol, and water (Vₜₒₗᵤₑₙₑ:Vₑₜₕₐₙₒₗ:V_{water} = (2-4):1:1) in a reaction vessel. The mixture is heated to 90-110 °C and reacted for 6-10 h. After the completion of the reaction, the resultant is cooled to room temperature. After the solid is completely precipitated, the resultant is subjected to suction filtration, and the filter cake is dried and recrystallized in 1,4-dioxane to give the compound of formula I.

Hal₁ is Cl, Br, or I.

The synthetic route of the P-type host material in the organic electroluminescent device described above is as follows:
When L₁ is not a linking bond, the synthetic route is:

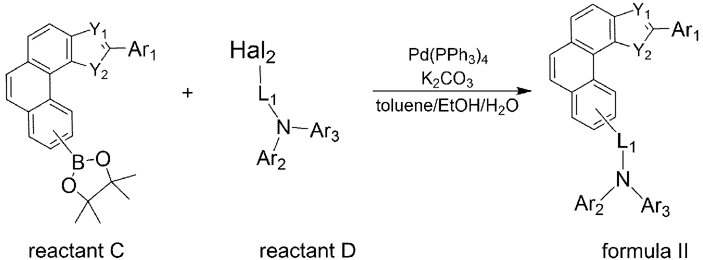

Under N₂ atmosphere, reactant C (1.0 eq), reactant D (1.0-1.2 eq), tetrakis(triphenylphosphine)palladium(0) (0.01-0.04 eq), and potassium carbonate (2.0-2.4 eq) are separately added to a mixed solvent of toluene, ethanol, and water (Vₜₒₗᵤₑₙₑ:Vₑₜₕₐₙₒₗ:V_{water} = (2-4):1:1) in a reaction vessel. The mixture is heated to 90-110 °C and reacted for 6-10 h. After the completion of the reaction, the resultant is cooled to room temperature. After the solid is completely precipitated, the resultant is subjected to suction filtration, and the filter cake is dried and recrystallized in 1,4-dioxane to give the compound of formula II.

When L₁ is a linking bond, the synthetic route is:

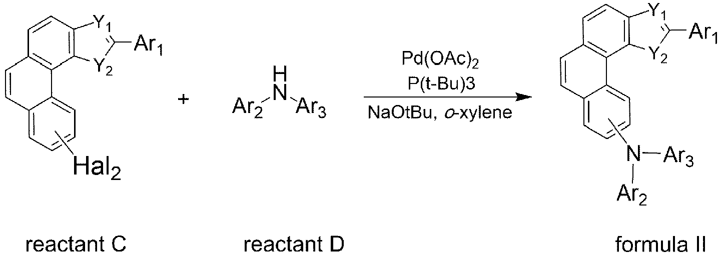

Under N₂ atmosphere, reactant C (1.0 eq), reactant D (1.0-1.2 eq), Pd(OAc)₂ (0.01-0.04 eq), P(t-Bu)₃ (0.05-0.1 eq), NaOtBu (2.0-2.5 eq), and o-xylene are added to a reaction vessel and dissolved. The mixture is heated to 135-145 °C and reacted for 2-4 h. After the completion of the reaction, the organic layer is extracted with ethyl acetate and separated by column chromatography to give the compound of formula II.

Hal₂ is Cl, Br, or I.

The synthetic route of the N-type host material in the organic electroluminescent device described above is as follows:

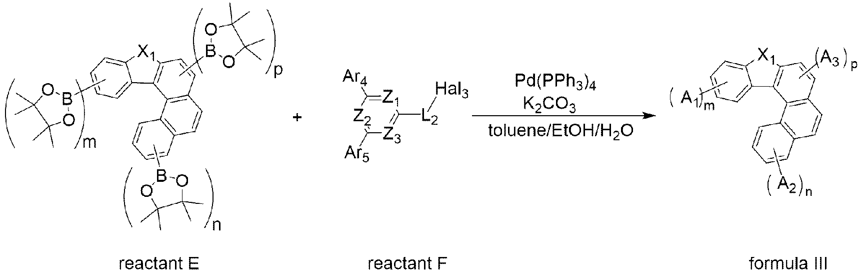

Under N₂ atmosphere, reactant E (1.0 eq), reactant F (1.0-1.2 eq), tetrakis(triphenylphosphine)palladium(0) (0.01-0.04 eq), and potassium carbonate (2.0-2.4 eq) are separately added to a mixed solvent of toluene, ethanol, and water (Vₜₒₗᵤₑₙₑ:Vₑₜₕₐₙₒₗ:V_{water} = (2-4):1:1) in a reaction vessel. The mixture is heated to 90-110 °C and reacted for 6-10 h. After the completion of the reaction, the resultant is cooled to room temperature. After the solid is completely precipitated, the resultant is subjected to suction filtration, and the filter cake is dried and recrystallized in 1,4-dioxane to give the compound of formula III.

Hal₃ is Cl, Br, or I.

### Example 1: Synthesis of the Second Hole Transport Layer material HT-13:

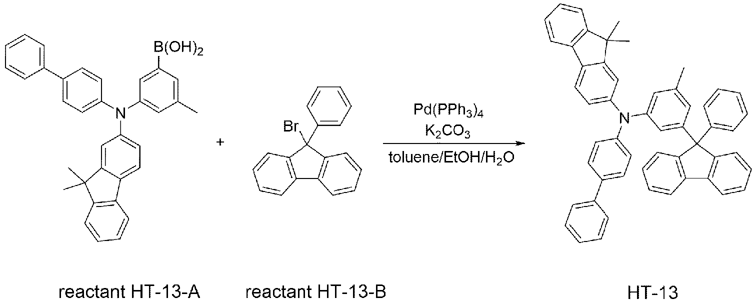

### CAS: HT-13-B: 55135-66-5

Under N₂ atmosphere, reactant HT-13-A (40 mmol), reactant HT-13-B (48 mmol), tetrakis(triphenylphosphine)palladium(0) (0.04 mmol), and potassium carbonate (88 mmol) were separately added to a mixed solvent of toluene (90 mL), ethanol (30 mL), and water (30 mL) in a reaction vessel. The mixture was heated to 100 °C and reacted for 8 h. After the completion of the reaction, the resultant was cooled to room temperature. After the solid was completely precipitated, the resultant was subjected to suction filtration, and the filter cake was dried and recrystallized in 1,4-dioxane to give compound HT-13 (24.37 g, yield: 88%).

### Characterization:

HPLC purity: > 99.8%;
mass spectrometry test: calculated 691.92; found 692.26;
elemental analysis:
   calculated: C, 92.00; H, 5.97; N, 2.02;
   found: C, 91.70; H, 6.25; N, 2.14.

### Example 2: Synthesis of the Second Hole Transport Layer material HT-24:

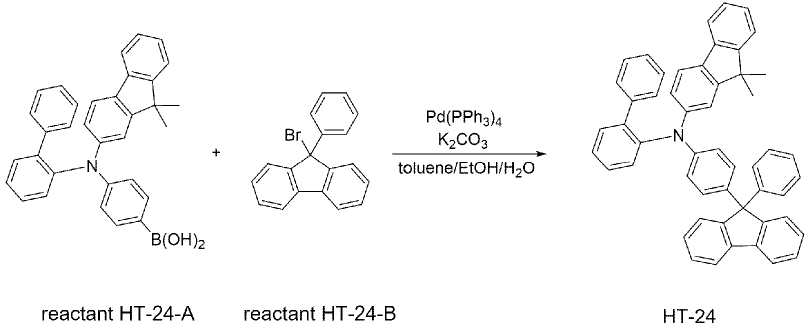

CAS: HT-24-A: 1959599-90-6
CAS: HT-24-B: 55135-66-5
CAS: HT-24: 2227490-15-3

Under N₂ atmosphere, reactant HT-24-A (40 mmol), reactant HT-24-B (48 mmol), tetrakis(triphenylphosphine)palladium(0) (0.04 mmol), and potassium carbonate (88 mmol) were separately added to a mixed solvent of toluene (90 mL), ethanol (30 mL), and water (30 mL) in a reaction vessel. The mixture was heated to 100 °C and reacted for 8 h. After the completion of the reaction, the resultant was cooled to room temperature. After the solid was completely precipitated, the resultant was subjected to suction filtration, and the filter cake was dried and recrystallized in 1,4-dioxane to give compound HT-24 (24.37 g, yield: 85%).

### Characterization:

HPLC purity: > 99.7%;
mass spectrometry test: calculated 677.89; found 678.30;
elemental analysis:
   calculated: C, 92.13; H, 5.80; N, 2.07;
   found: C, 91.93; H, 6.02; N, 2.15.

### Example 3: Synthesis of P-Type Host Material RH1-32:

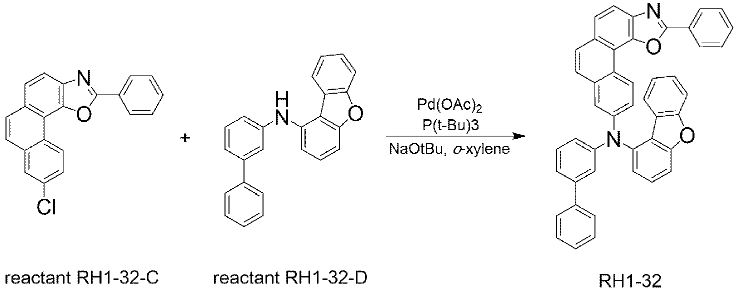

CAS: RH1-32-C: 2085325-16-0
CAS: RH1-32-D: 2230816-66-5

Under N₂ atmosphere, reactant RH1-32-C (40 mmol), reactant RH1-32-D (44 mmol), Pd(OAc)₂ (0.4 mmol), P(t-Bu)₃ (2 mmol), NaOtBu (88 mmol), and o-xylene were added to a reaction vessel and dissolved. The mixture was heated to 140 °C and reacted for 4 h. After the completion of the reaction, the organic layer was extracted with ethyl acetate and separated by column chromatography to give compound RH1-32 (19.62 g, yield: 78%).

### Characterization:

HPLC purity: > 99.7%;
mass spectrometry test: calculated 628.73; found 629.01;
elemental analysis:
   calculated: C, 85.97; H, 4.49; N, 4.46; O, 5.09;
   found: C, 85.82; H, 4.62; N, 4.52; O, 5.16.

### Example 4: Synthesis of P-Type Host Material RH1-50:

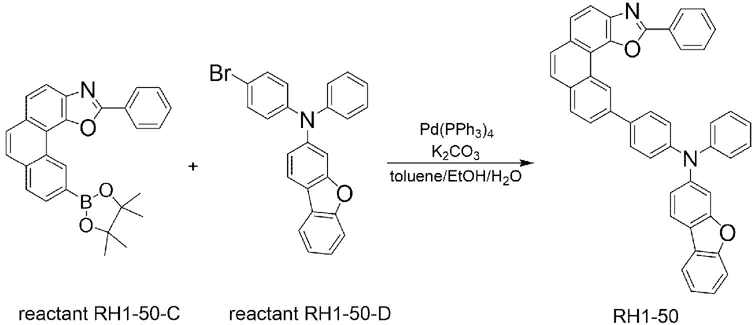

CAS: RH1-50-C: 2085325-18-2
CAS: RH1-50-D: 2222194-15-0
CAS: RH1-50: 2649505-53-1

Under N₂ atmosphere, reactant RH1-50-C (40 mmol), reactant RH1-50-D (48 mmol), tetrakis(triphenylphosphine)palladium(0) (0.6 mmol), and potassium carbonate (84 mmol) were separately added to a mixed solvent of toluene (90 mL), ethanol (30 mL), and water (30 mL) in a reaction vessel. The mixture was heated to 95 °C and reacted for 10 h. After the completion of the reaction, the resultant was cooled to room temperature. After the solid was completely precipitated, the resultant was subjected to suction filtration, and the filter cake was dried and recrystallized in 1,4-dioxane to give compound RH1-50 (20.13 g, yield: 80%).

### Characterization:

HPLC purity: > 99.8%;
mass spectrometry test: calculated 628.73; found 629.01;
elemental analysis:
   calculated: C, 85.97; H, 4.49; N, 4.46; O, 5.09;
   found: C, 85.82; H, 4.62; N, 4.52; O, 5.16.

### Example 5: Synthesis of P-Type Host Material RH1-57:

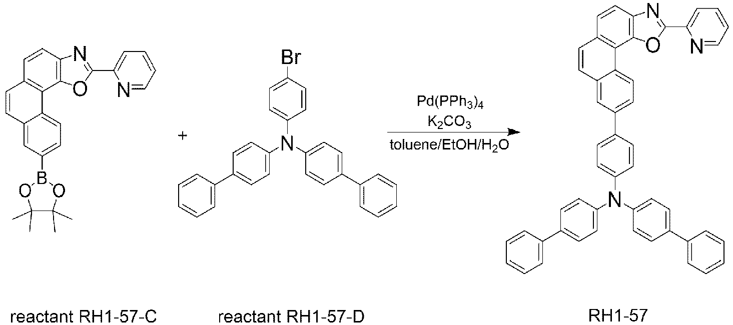

Under N₂ atmosphere, reactant RH1-57-C (40 mmol), reactant RH1-57-D (45 mmol), tetrakis(triphenylphosphine)palladium(0) (0.4 mmol), and potassium carbonate (84 mmol) were separately added to a mixed solvent of toluene (90 mL), ethanol (30 mL), and water (30 mL) in a reaction vessel. The mixture was heated to 95 °C and reacted for 8 h. After the completion of the reaction, the mixture was cooled to room temperature. After the solid was completely precipitated, the resultant was subjected to suction filtration, and the filter cake was dried and recrystallized in 1,4-dioxane to give compound RH1-57 (22.98 g, yield: 83%).

### Characterization:

HPLC purity: > 99.8%;
mass spectrometry test: calculated 691.83; found 692.19;
elemental analysis:
   calculated: C, 86.81; H, 4.81; N, 6.07; O, 2.31;
   found: C, 86.58; H, 4.98; N, 6.12; O, 2.39.

### Example 6: Synthesis of N-Type Host Material RH2-5:

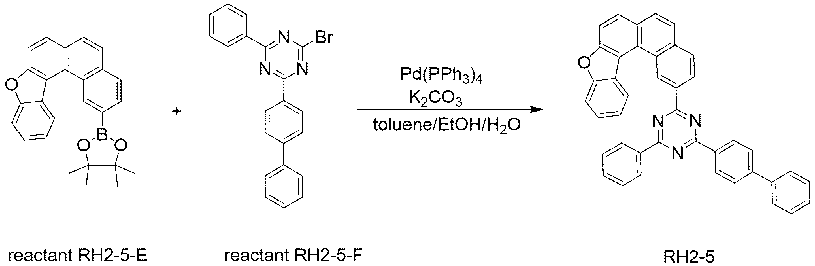

CAS: RH2-5-E: 2204284-97-7
CAS: RH2-5-F: 1970122-69-0
CAS: RH2-5: 2649505-87-1

Under N₂ atmosphere, reactant RH2-5-E (40 mmol), reactant RH2-5-F (48 mmol), tetrakis(triphenylphosphine)palladium(0) (0.4 mmol), and potassium carbonate (84 mmol) were separately added to a mixed solvent of toluene (90 mL), ethanol (30 mL), and water (30 mL) in a reaction vessel. The mixture was heated to 95 °C and reacted for 8 h. After the completion of the reaction, the resultant was cooled to room temperature. After the solid was completely precipitated, the resultant was subjected to suction filtration, and the filter cake was dried and recrystallized in 1,4-dioxane to give compound RH2-5 (17.28 g, yield: 75%).

### Characterization:

HPLC purity: > 99.7%;
mass spectrometry test: calculated 575.67; found 576.13;
elemental analysis:
   calculated: C, 85.54; H, 4.38; N, 7.30; O, 2.78;
   found: C, 85.29; H, 4.62; N, 7.35; O, 2.84.

### Example 7: Synthesis of N-Type Host Material RH2-29:

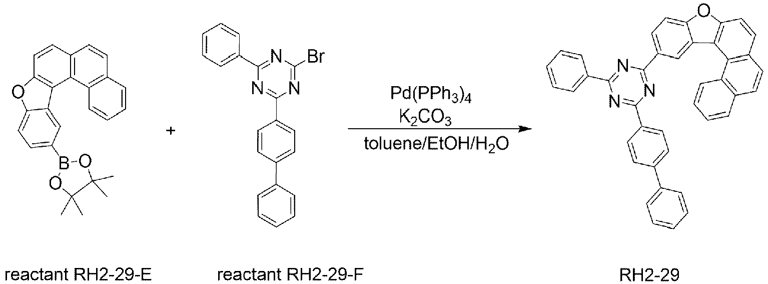

### CAS: RH2-29: 2649507-42-4

Under N₂ atmosphere, reactant RH2-29-E (40 mmol), reactant RH2-29-F (48 mmol), tetrakis(triphenylphosphine)palladium(0) (0.4 mmol), and potassium carbonate (84 mmol) were separately added to a mixed solvent of toluene (90 mL), ethanol (30 mL), and water (30 mL) in a reaction vessel. The mixture was heated to 90 °C and reacted for 8 h. After the completion of the reaction, the resultant was cooled to room temperature. After the solid was completely precipitated, the resultant was subjected to suction filtration, and the filter cake was dried and recrystallized in 1,4-dioxane to give compound RH2-29 (18.20 g, yield: 79%).

### Characterization:

HPLC purity: > 99.7%;
mass spectrometry test: calculated 575.67; found 576.07;
elemental analysis:
   calculated: C, 85.54; H, 4.38; N, 7.30; O, 2.78;
   found: C, 85.39; H, 4.50; N, 7.36; O, 2.84.

### Example 8: Synthesis of N-Type Host Material RH2-78:

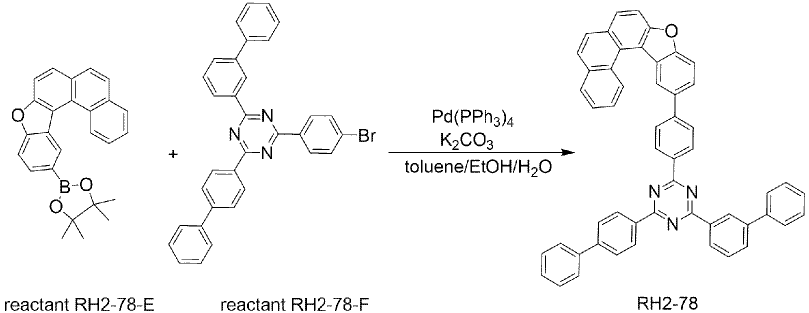

Under N₂ atmosphere, reactant RH2-78-E (40 mmol), reactant RH2-78-F (48 mmol), tetrakis(triphenylphosphine)palladium(0) (0.4 mmol), and potassium carbonate (84 mmol) were separately added to a mixed solvent of toluene (90 mL), ethanol (30 mL), and water (30 mL) in a reaction vessel. The mixture was heated to 95 °C and reacted for 8 h. After the completion of the reaction, the resultant was cooled to room temperature. After the solid was completely precipitated, the resultant was subjected to suction filtration, and the filter cake was dried and recrystallized in 1,4-dioxane to give compound RH2-78 (17.28 g, yield: 72%).

### Characterization:

HPLC purity: > 99.8%;
mass spectrometry test: calculated 727.87; found 728.23;
elemental analysis:
   calculated: C, 87.46; H, 4.57; N, 5.77; O, 2.20;
   found: C, 87.72; H, 4.67; N, 5.84; O, 2.27.

In the present disclosure, the organic electroluminescent device has a high turn-on voltage and also has a relatively low driving voltage, as well as has the advantages of high efficiency and long service life of a dual-host red-light device to alleviate a phenomenon of the color reddening caused by crosstalk, wherein the organic electroluminescent device is prepared by a specific combination of a specific arylamine derivative represented by formula I of the present disclosure contained in a hole transport region and a dual-host red-light material represented by formulae II and III of the present disclosure contained in a light emitting layer.

The organic electroluminescent device of the present disclosure comprises a first electrode, a second electrode facing the first electrode, a light emitting layer between the first electrode and the second electrode, and a hole transport region between the first electrode and the light emitting layer.

The hole transport region of the present disclosure comprises a hole injection layer, a first hole transport layer, and a second hole transport layer. A second hole transport layer material may also be used as a light emitting auxiliary layer material and an electron blocking layer material. The second hole transport layer material of the present disclosure is composed of the compound represented by formula I.

The host material of the present disclosure is a red-light dual-host material, which is composed of the compounds represented by formulae II and III.

The organic electroluminescent apparatus of the present disclosure may be of a top emission type, a bottom emission type, or a dual emission type. The device described herein may be used in an organic light-emitting device, an organic solar cell, an electronic paper, an organic photoreceptor, or an organic thin-film transistor.

The organic electroluminescent apparatus of the present disclosure comprises a first electrode, a second electrode facing the first electrode, a light emitting layer between the first electrode and the second electrode, a hole transport region between the first electrode and the light emitting layer, and an electron transport region between the light emitting layer and the second electrode. One of the first electrode and the second electrode is an anode, and the other is a cathode.

The organic light-emitting element may have a structure comprising a hole injection layer, a hole transport layer, an electron blocking layer, a second hole transport layer, a light emitting layer, a hole blocking layer, an electron transport layer, an electron injection layer, and the like as an organic layer. However, the structure of the organic light-emitting element is not limited thereto, and may comprise less or more organic layers.

The organic light-emitting element may be manufactured by sequentially stacking the first electrode, the organic layer, and the second electrode on a substrate. The substrate may be manufactured by physical vapor deposition (PVD) methods such as sputtering or ebeam evaporation.

A metal, or a metal oxide with conductivity, or an alloy thereof is evaporated on a substrate to form a first electrode, an organic layer comprising a hole injection layer, a hole transport layer, a light emitting layer, and an electron transport layer is formed on the first electrode, and a substance which may be used as a second electrode is evaporated on the organic layer. In addition to such a method, the second electrode substance, the organic layer, and the first electrode substance may be sequentially evaporated on the substrate to manufacture the organic light-emitting element.

In addition, for the compounds represented by formulae I, II, and III described above, not only a vacuum evaporation method but also a solution coating method may be used to form an organic layer when manufacturing an organic light-emitting element. The solution coating method refers to, but is not limited to, spin coating, dip coating, blade coating, inkjet printing, screen printing, spraying, roll coating, and the like.

In the present disclosure, the first electrode is an anode, and the second electrode is a cathode.

Generally, the anode substance is preferably a substance having a high work function in order to smoothly inject holes into the organic layer. In the present disclosure, specific examples of the anode substance include metals such as vanadium, chromium, copper, zinc, and gold, or alloys thereof; metal oxides such as zinc oxide, indium oxide, indium tin oxide (ITO), and indium zinc oxide (IZO); combinations of metals and oxides such as ZnO:Al or SnO₂:Sb; conductive polymers such as polypyrrole and polyaniline; and the like.

Generally, the cathode substance described above is preferably a substance having a low work function in order to easily inject electrons into the organic layer. Specific examples of the cathode substance include metals such as magnesium, calcium, sodium, potassium, titanium, indium, yttrium, lithium, gadolinium, aluminum, silver, tin, and lead, or alloys thereof; multi-layer substances such as LiF/A1 or LiO₂/Al and Mg/Ag, and the like.

The hole transport region refers to a region in which holes are transported between the first electrode and the light emitting layer. The hole transport region is a region that receives holes from the hole injection layer and transports the holes to the light emitting layer. The hole transport region may be placed between the anode (or hole injection layer) and the light emitting layer. The hole transport region may be used to smoothly move holes transferred from the anode to the light emitting layer and block electrons transferred from the cathode to allow the electrons to remain in the light emitting layer.

The hole transport region of the present disclosure comprises a hole injection layer, a first hole transport layer, and a second hole transport layer. The second hole transport layer material may also be used as an emission auxiliary layer material and an electron blocking layer material.

The second hole transport layer material of the present disclosure is composed of the compound represented by formula I.

In the present disclosure, the hole injection layer is preferably a p-doped hole injection layer, which refers to a hole injection layer doped with a p-dopant. The p-dopant is a material capable of imparting p-type semiconductor characteristics. The p-type semiconductor characteristics refer to characteristics of injecting holes or transporting holes at a HOMO energy level, that is, characteristics of a material having high hole conductivity.

The second hole transport layer may be placed between the anode and the light emitting layer, or between the cathode and the light emitting layer. When the second hole transport layer is placed between the anode and the light emitting layer, it may be used to facilitate hole injection and/or hole transport, or to prevent electron overflow. When the second hole transport layer is placed between the cathode and the light emitting layer, it may be used to facilitate electron injection and/or electron transport, or to prevent hole overflow.

A light-emitting substance in the light emitting layer is a substance capable of receiving holes and electrons from the hole transport layer and the electron transport layer and combining them to emit light in the visible light region, and is preferably a substance having high quantum efficiency for fluorescence or phosphorescence.

The light emitting layer may comprise a host material and a dopant material.

The host material of the present disclosure is a red-light dual-host material, which is composed of the compounds represented by formulae II and III.

The electron transport region may comprise at least one of an electron buffer layer, a hole blocking layer, an electron transport layer, and an electron injection layer, preferably at least one of an electron transport layer and an electron injection layer. The electron transport region is a layer capable of improving degradation of the luminance due to a change in current characteristics in an apparatus when the apparatus is exposed to a high temperature during the process of manufacturing a panel, and it may control charge flow characteristics.

There is no particular limitation on the materials of the other layers in the OLED device except for the specific combination of the compound of formula I contained in the second hole transport layer and the compounds of formulae II and III contained in the light emitting layer disclosed herein. Existing hole injection materials, hole transport materials, dopant materials, hole blocking layer materials, electron transport layer materials, and electron injection materials may be used.

Examples of the hole injection layer materials include metalloporphyrin, oligothiophene, arylamine derivatives, hexanitrilehexaazatriphenylene-based organic substances, quinacridone-based organic substances, perylene-based organic substances, anthraquinone, polyaniline, polythiophene-based conductive polymers, and the like. The P-dopant for P doping may be exemplified by the following compounds, but is not limited thereto.

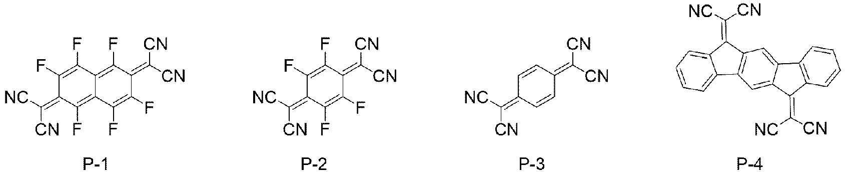

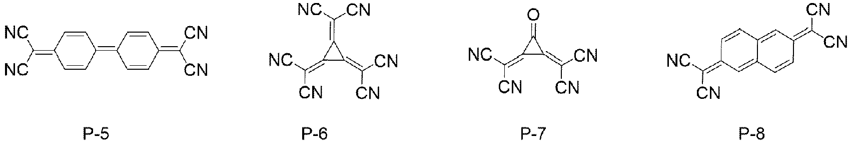

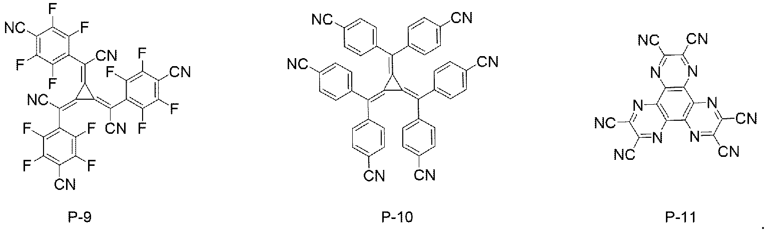

A first hole transport material may be selected from arylamine derivatives, conductive polymers, block copolymers having both a conjugated portion and a non-conjugated portion, and the like. Specifically, the first hole transport layer material is selected from the following compounds, but is not limited thereto.

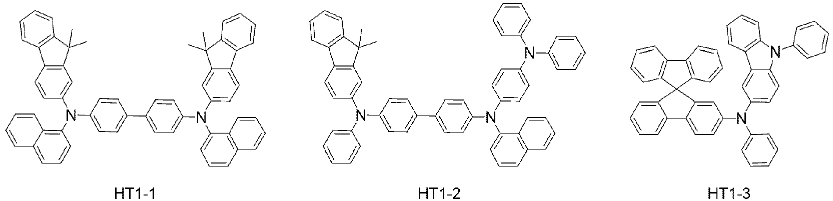

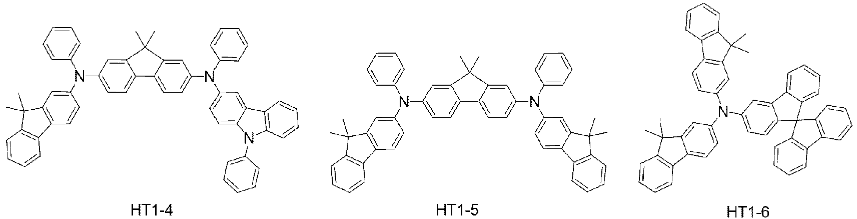

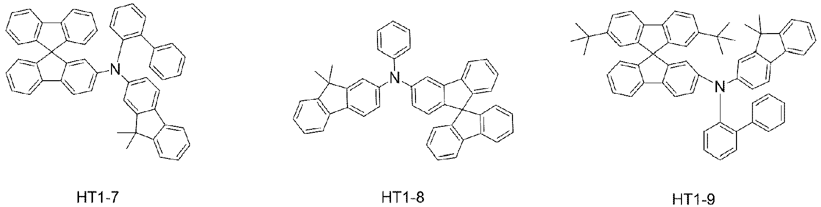

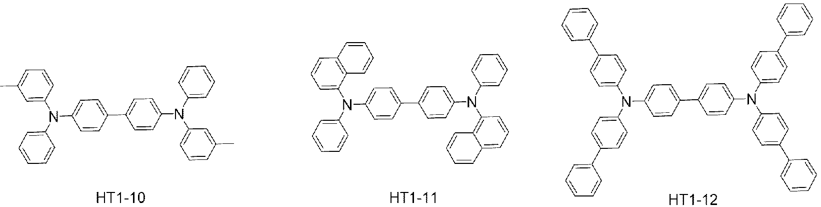

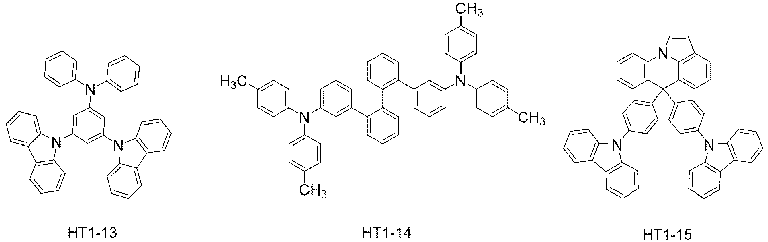

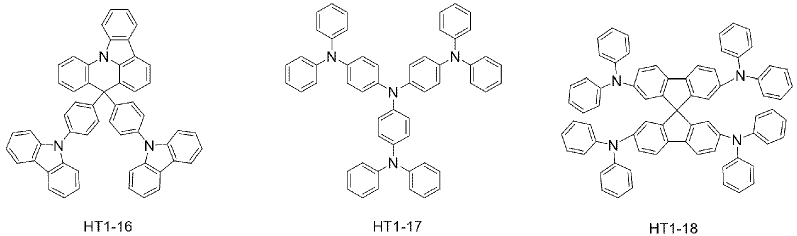

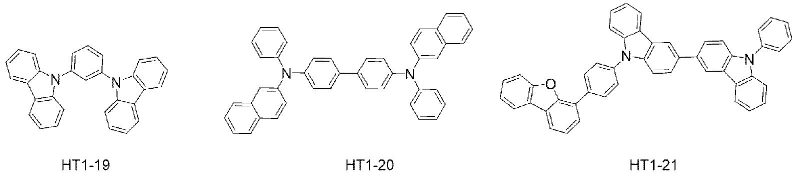

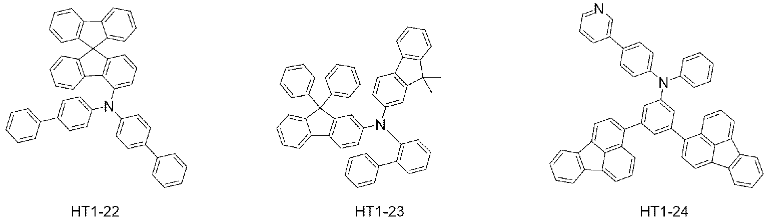

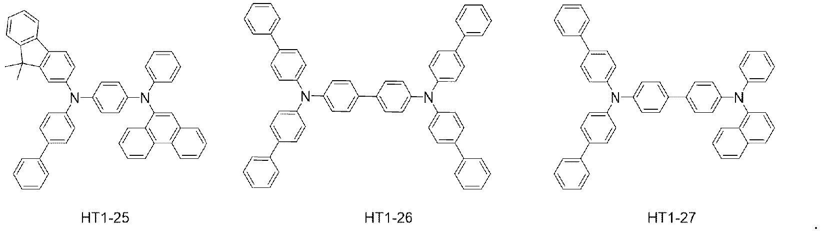

The second hole transport layer of the present disclosure is formed from the compound represented by general formula I.

The red-light dual-host material of the present disclosure is the compounds represented by general formula II and general formula III.

The dopant material of the present disclosure is selected from red-light dopant materials, including aromatic amine derivatives, styrylamine compounds, boron complexes, fluoranthene compounds, metal complexes, and the like. Specifically, the red-light dopant material of the present disclosure is selected from the following compounds, but is not limited thereto.

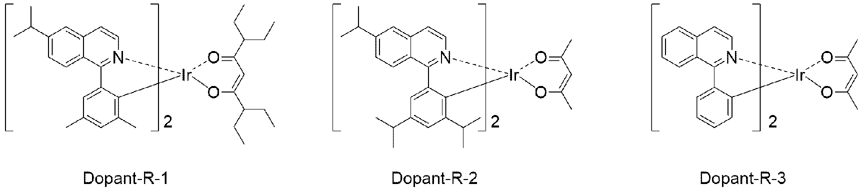

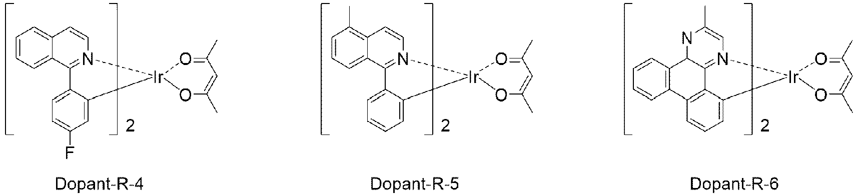

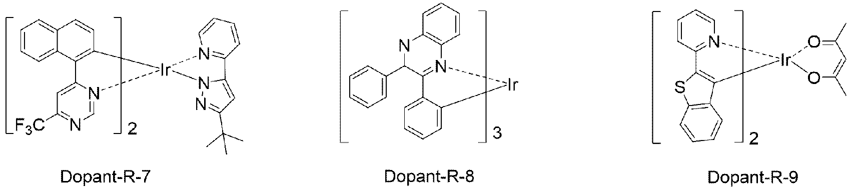

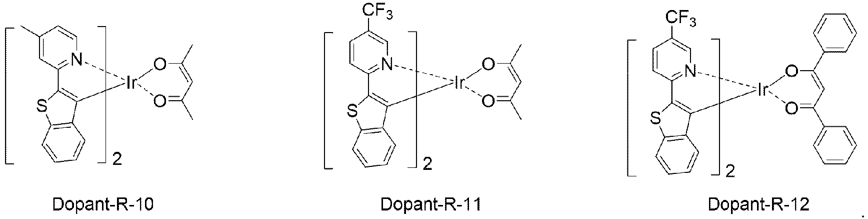

The electron transport layer (hole blocking layer) material is derivatives such as oxazole, imidazole, thiazole, or triazine, metal chelates, quinoline derivatives, quinoxaline derivatives, diazanthracene derivatives, diazaphenanthrene derivatives, silicon-containing heterocyclic compounds, perfluorinated oligomers, and the like. Specifically, the electron transport layer material is selected from the following compounds, but is not limited thereto.

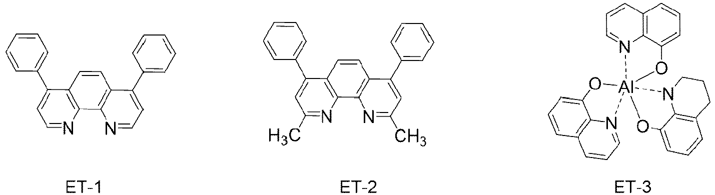

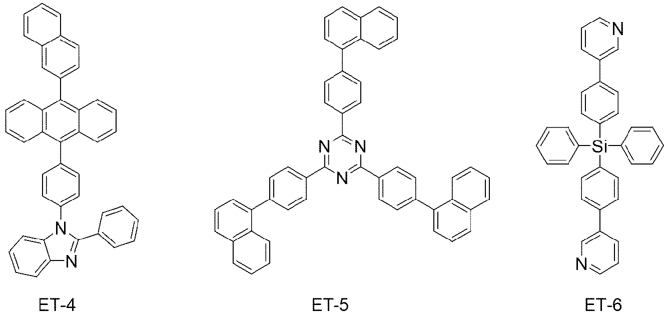

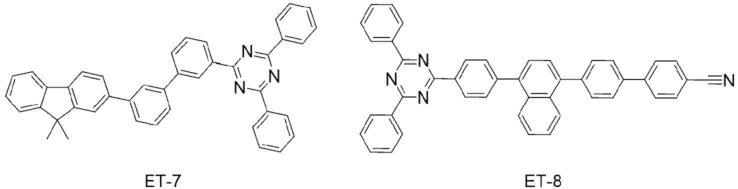

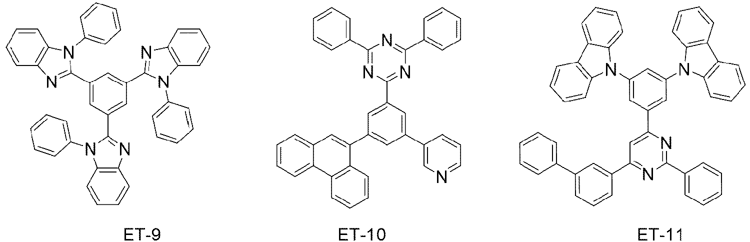

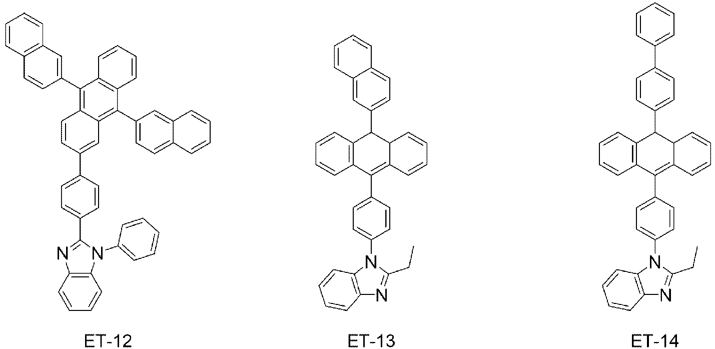

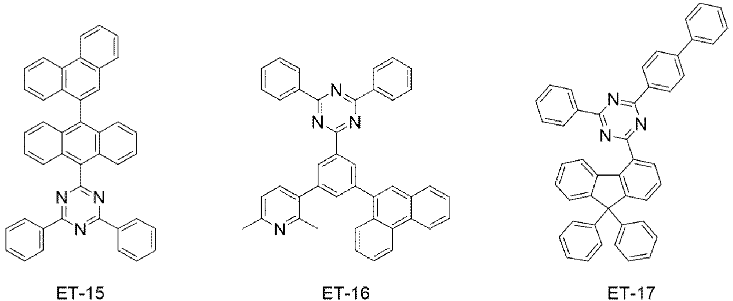

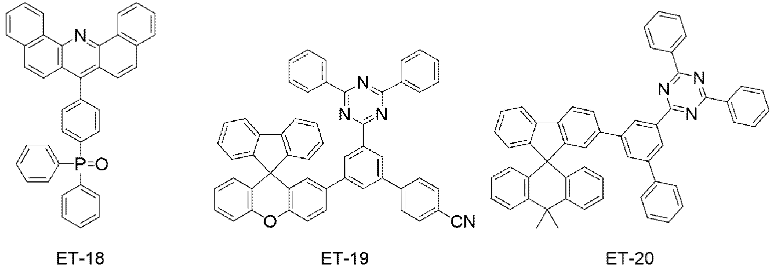

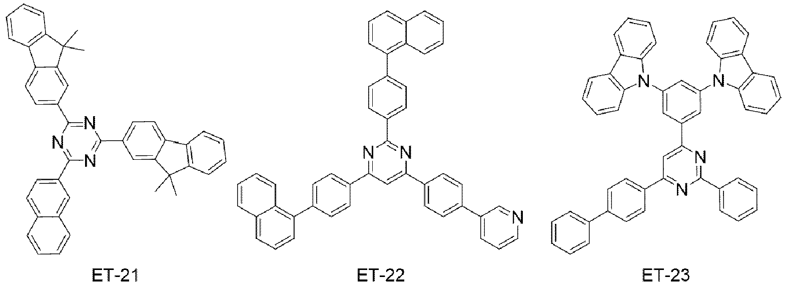

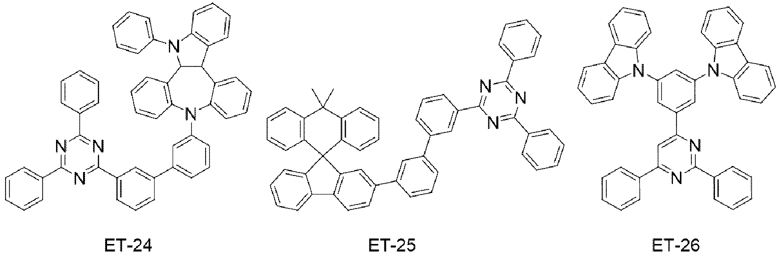

The electron injection layer material includes, but is not limited to, oxazole, oxadiazole, triazole, imidazole, perylene tetracarboxylic acid, fluorenylidene methane, anthrone, and derivatives thereof; metals such as magnesium, calcium, sodium, potassium, titanium, indium, yttrium, lithium, gadolinium, aluminum, silver, tin, and ytterbium, or alloys thereof; metal complexes; nitrogen-containing 5-membered ring derivatives; or the like.

The organic electroluminescent composition and the organic electroluminescent device provided by the present disclosure are specifically described below with reference to specific examples.

A preparation method for a red-light organic electroluminescent device comprises the following steps:
a. ITO anode: an ITO (indium tin oxide)-Ag-ITO (indium tin oxide) glass substrate with a coating thickness of 150 nm was washed 2 times in distilled water, ultrasonically washed for 30 min, repeatedly washed 2 times in distilled water, and ultrasonically washed for 10 min. After washing, the substrate was transferred to a spin dryer for spin drying, baked in a vacuum oven at 220 °C for 2 h, and cooled after baking for use. The substrate was taken as an anode, and other functional layers were sequentially evaporated on the substrate by performing a device evaporation process with an evaporation machine;
b. HIL (hole injection layer): hole injection layer materials HT1-12 and P-9 having the chemical formulae shown as follows were vacuum-evaporated at an evaporation rate of 1 Å/s; the ratio of the evaporation rate of HT1-12 to the evaporation rate of P-9 was 97:3, and the thickness was 10 nm;
c. HTL (hole transport layer): HT1-12 with a thickness of 125 nm was vacuum-evaporated as a hole transport layer on the hole injection layer at an evaporation rate of 1.0 Å/s;
d. second hole transport layer: the compound represented by formula I provided herein with a thickness of 90 nm was vacuum-evaporated as a second hole transport layer on the hole transport layer at an evaporation rate of 1.0 Å/s;
e. EML (light emitting layer): host materials (Host) represented by formula II and formula III and a dopant material (Dopant-R-1) (the chemical formulae of Host and Dopant are shown as follows) with a total thickness of 40 nm were vacuum-evaporated as a light emitting layer on the second hole transport layer described above at an evaporation rate of 1 Å/s, wherein the evaporation ratio of the host materials represented by formulae II and III was 4:6, and the ratio of the evaporation rate of Host to the evaporation rate of Dopant-R-1 was 97:3;
f. HBL (hole blocking layer): a hole blocking layer HB with a thickness of 5.0 nm was vacuum-evaporated at an evaporation rate of 0.5 Å/s;
g. ETL (electron transport layer): ET-10 and Liq with a thickness of 30 nm were vacuum-evaporated as an electron transport layer at an evaporation rate of 1 Å/s, wherein the ratio of the evaporation rate of ET-10 to the evaporation rate of Liq was 1:1;
h. EIL (electron injection layer): a Yb film layer with a thickness of 1.0 nm was evaporated at an evaporation rate of 0.5 Å/s to form an electron injection layer;
i. cathode: magnesium and silver with a thickness of 18 nm were evaporated at an evaporation rate ratio of 1 Å/s to form a cathode, and the ratio of the evaporation rate of magnesium to the evaporation rate of silver was 1:9;
j. light extraction layer: CPL with a thickness of 70 nm was vacuum-evaporated as a light extraction layer on the cathode at an evaporation rate of 1 Å/s; and
k. encapsulation of evaporated substrate; firstly, the cleaned cover plate was coated with an UV glue by using a gluing device, the coated cover plate was moved to a pressing working section, the evaporated substrate was placed on the top of the cover plate, and finally, the substrate and the cover plate were laminated under the action of a laminating device, while the UV glue was irradiated and cured.

The structure of the device is as follows: ITO/Ag/ITO/HT1-12:P-9 (10 nm)/HT1-12 (125 nm)/formula I (90 nm)/(formula II + formula III):Dopant-R-1 (40 nm)/HB (5 nm)/ET-10:Liq (30 nm)/Yb (1 nm)/Mg:Ag (18 nm)/CPL (70 nm).

**Table 1. Materials required for the layers**

| | |
|---|---|
| Hole injection layer | 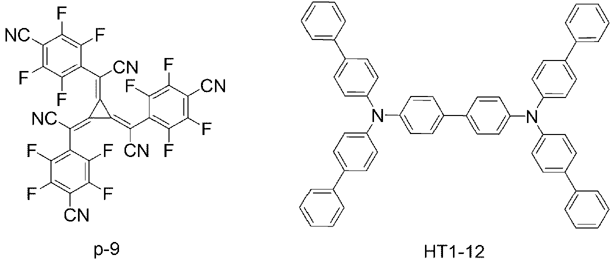 |
| Hole transport layer | 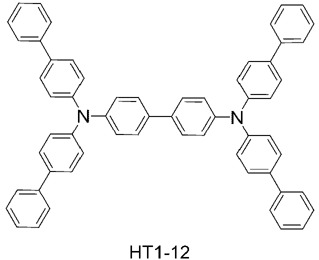 |
| Second hole transport layer | 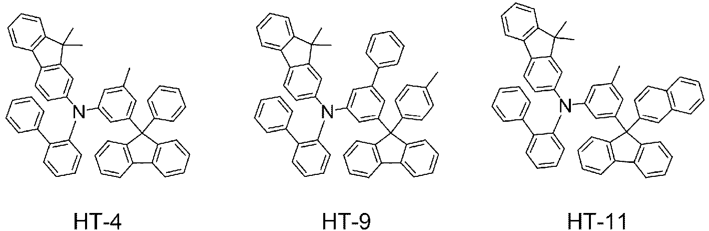 |
| | 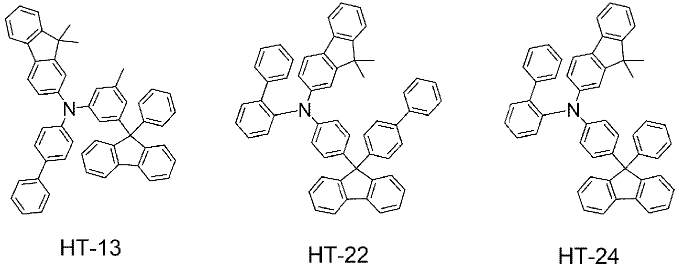 |
| | 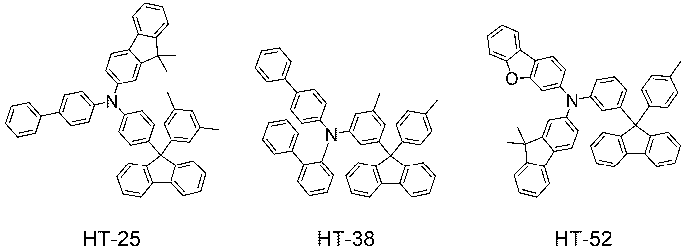 |
| | 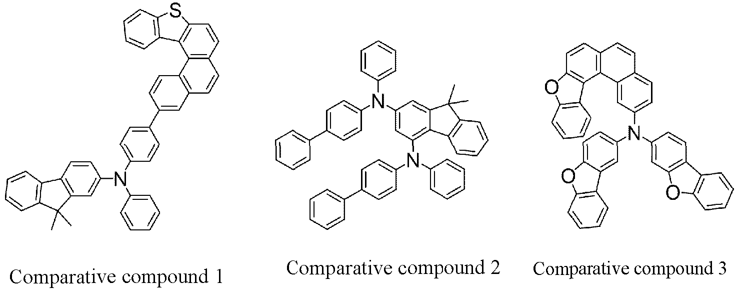 |
| light emitting layer | 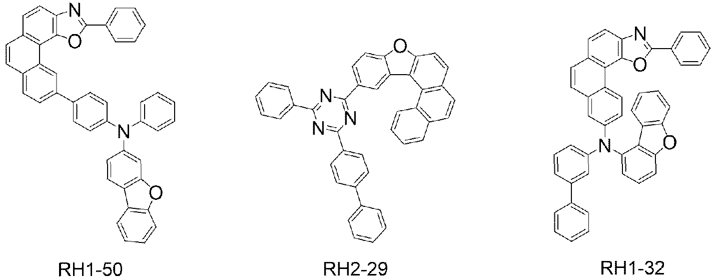 |
| | 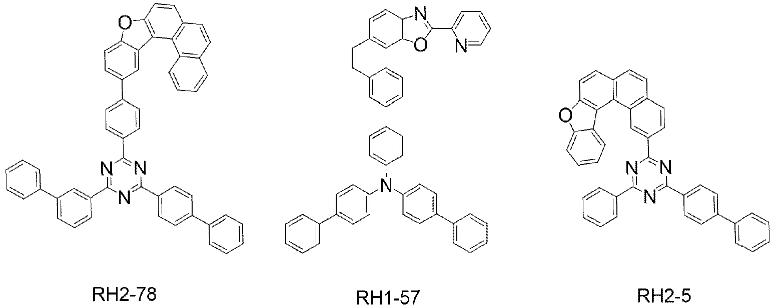 |
| | 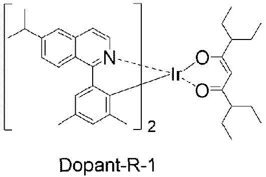 |
| Hole blocking layer | 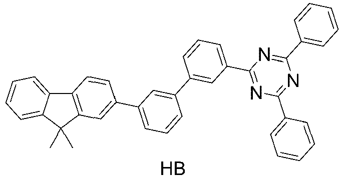 |
| Electron transport layer | 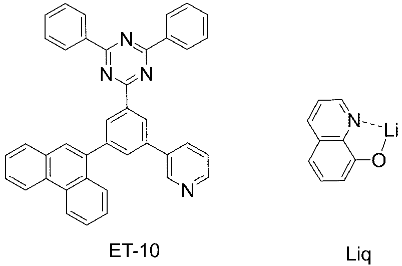 |
| Light extraction layer | 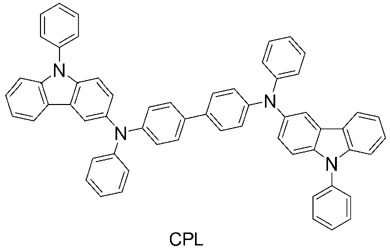 |

### Application Examples 1-9

The organic electroluminescent devices of Application Examples 1-9 were prepared according to the preparation method for the organic electroluminescent devices described above. The compounds RH1-50 and RH2-29 shown in Table 1 were used as host materials, and HT-4, HT-9, HT-11, HT-13, HT-22, HT-24, HT-25, HT-38, and HT-52 were used as second hole transport layer materials.

### Application Example 10

The organic electroluminescent device of Application Example 10 was prepared according to the preparation method for the organic electroluminescent devices described above. The compounds RH1-32 and RH2-78 shown in Table 1 were used as host materials, and HT-13 was used as a second hole transport layer material.

### Application Example 11

The organic electroluminescent device of Application Example 11 was prepared according to the preparation method for the organic electroluminescent devices described above. The compounds RH1-57 and RH2-5 shown in Table 1 were used as host materials, and HT-13 was used as a second hole transport layer material.

### Application Example 12

The organic electroluminescent device of Application Example 12 was prepared according to the preparation method for the organic electroluminescent devices described above. The compounds RH1-32 and RH2-78 shown in Table 1 were used as host materials, and HT-24 was used as a second hole transport layer material.

### Application Example 13

The organic electroluminescent device of Application Example 13 was prepared according to the preparation method for the organic electroluminescent devices described above. The compounds RH1-57 and RH2-5 shown in Table 1 were used as host materials, and HT-24 was used as a second hole transport layer material.

### Comparative Examples 1-3

The organic electroluminescent devices of Comparative Examples 1-3 were prepared according to the preparation method for the organic electroluminescent devices described above. The compounds RH1-50 and RH2-29 shown in Table 1 were used as host materials, and comparative compounds 1-3 were used as second hole transport layer materials.

### Comparative Example 4

The organic electroluminescent device of Comparative Example 4 was prepared according to the preparation method for the organic electroluminescent devices described above. The compounds RH1-32 and RH2-78 shown in Table 1 were used as host materials, and comparative compound 2 was used as a second hole transport layer material.

### Comparative Example 5

The organic electroluminescent device of Comparative Example 5 was prepared according to the preparation method for the organic electroluminescent devices described above. The compounds RH1-57 and RH2-5 shown in Table 1 were used as host materials, and comparative compound 2 was used as a second hole transport layer material.

The driving voltages and the turn-on voltages of the organic electroluminescent devices obtained in Device Application Examples 1-13 and Device Comparative Examples 1-5 described above were tested at a luminance of 6000 (nits), and the test results are shown in Table 2 and FIG. 1.

**Table 2. Test results (luminance value is 6000 nits)**

| Example | Second hole transport layer | Host 1 | Host 2 | **Turn-on** voltage (V) | Driving voltage (V) |
|---|---|---|---|---|---|
| Application Example 1 | HT-4 | RH1-50 | RH2-29 | 2.22 | 3.43 |
| Application Example 2 | HT-9 | RH1-50 | RH2-29 | 2.21 | 3.43 |
| Application Example 3 | HT-11 | RH1-50 | RH2-29 | 2.23 | 3.41 |
| Application Example 4 | HT-13 | RH1-50 | RH2-29 | 2.26 | 3.45 |
| Application Example 5 | HT-22 | RH1-50 | RH2-29 | 2.20 | 3.44 |
| Application Example 6 | HT-24 | RH1-50 | RH2-29 | 2.24 | 3.46 |
| Application Example 7 | HT-25 | RH1-50 | RH2-29 | 2.22 | 3.43 |
| Application Example 8 | HT-38 | RH1-50 | RH2-29 | 2.23 | 3.44 |
| Application Example 9 | HT-52 | RH1-50 | RH2-29 | 2.23 | 3.43 |
| Application Example 10 | HT-13 | RH1-32 | RH2-78 | 2.24 | 3.45 |
| Application Example 11 | HT-13 | RH1-57 | RH2-5 | 2.22 | 3.42 |
| Application Example 12 | HT-24 | RH1-32 | RH2-78 | 2.24 | 3.43 |
| Application Example 13 | HT-24 | RH1-57 | RH2-5 | 2.22 | 3.44 |
| Comparative Example 1 | Comparative compound 1 | RH1-50 | RH2-29 | 2.13 | 3.50 |
| Comparative Example 2 | Comparative compound 2 | RH1-50 | RH2-29 | 2.14 | 3.46 |
| Comparative Example 3 | Comparative compound 3 | RH1-50 | RH2-29 | 2.11 | 3.52 |
| Comparative Example 4 | Comparative compound 2 | RH1-32 | RH2-78 | 2.12 | 3.51 |
| Comparative Example 5 | Comparative compound 2 | RH1-57 | RH2-5 | 2.13 | 3.49 |

In the present disclosure, the voltage of the red-light device at 6000 nits was used as the driving voltage.

The turn-on voltages and driving voltages obtained from Table 2 show that the turn-on voltage was increased by 0.1 V to 0.15 V, which has been a significant improvement in the art. In the present disclosure, the organic electroluminescent device prepared by the specific combination of the specific arylamine derivative represented by formula I of the present disclosure contained in the hole transport region and the dual-host red-light material represented by formulae II and III of the present disclosure contained in the light emitting layer is capable of improving the turn-on voltage and keeping the relatively low driving voltage.

It is obvious that the examples described above are merely illustrative for a clear explanation and are not intended to limit the embodiments. Various changes or modifications can be made by those of ordinary skills in the art on the basis of the above description. It is unnecessary and impossible to exhaustively list all the embodiments herein. Obvious changes or modifications derived therefrom still fall within the protection scope of the present disclosure.

## Claims

1. An organic electroluminescent device, comprising a first electrode, a second electrode facing the first electrode, a light emitting layer between the first electrode and the second electrode, and a hole transport region between the first electrode and the light emitting layer, wherein the hole transport region comprises a hole injection layer, a first hole transport layer, and a second hole transport layer;
the material of the second hole transport layer has a general structural formula shown as formula I: 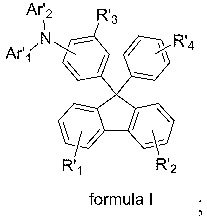 wherein
Ar'₁ and Ar'₂ are each independently substituted or unsubstituted C₆-C₂₄ aryl, or substituted or unsubstituted 3- to 24-membered heteroaryl;
R'₁-R'₃ are each independently hydrogen, deuterium, substituted or unsubstituted C₁-C₆ alkyl, substituted or unsubstituted C₆-C₁₈ aryl, substituted or unsubstituted 3- to 18-membered heteroaryl, substituted or unsubstituted C₃-C₁₂ cycloalkyl, or substituted or unsubstituted C₁-C₆ alkoxy;
R'₄ is hydrogen, methyl, ethyl, phenyl, or biphenyl;
R'₁-R'₄ are each independently bonded to or fused with respective phenyl rings;
the light emitting layer comprises a P-type host material and an N-type host material;
wherein the P-type host material has a general structural formula shown as formula II: 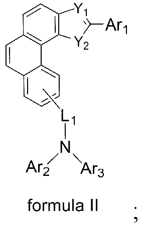 wherein
Y₁ and Y₂ are in accordance with one of the following conditions:
Y₁ is N, and Y₂ is O;
Y₁ is N, and Y₂ is S;
Y₁ is O, and Y₂ is N; and
Y₁ is S, and Y₂ is N;
L₁ is a linking bond, substituted or unsubstituted C₆-C₁₈ arylene, or substituted or unsubstituted 3- to 18-membered heteroarylene;
Ar₁-Ar₃ are each independently substituted or unsubstituted C₆-C₂₄ aryl, or substituted or unsubstituted 3- to 24-membered heteroaryl;
Ar₁-Ar₃ are each independently bonded to or fused with respective phenyl rings;
the N-type host material has a general structural formula shown as formula III: 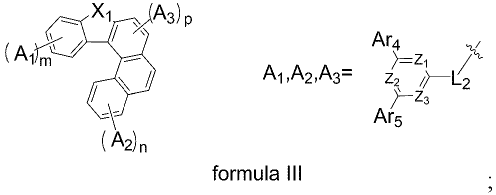 wherein
X₁ is selected from O or S;
one of m, n, and p is 1, and the rest are 0;
Z₁-Z₃ are each independently N or C, and at least one of Z₁-Z₃ is N;
Ar₄-Ar₅ are each independently substituted or unsubstituted C₆-C₂₄ aryl, or substituted or unsubstituted 3- to 24-membered heteroaryl; and
L₂ is a linking bond, substituted or unsubstituted C₆-C₁₈ arylene, or substituted or unsubstituted 3- to 18-membered heteroarylene.

2. The organic electroluminescent device of claim 1, wherein the material of the second hole transport layer is selected from one of the following formulae I-1 to I-3: 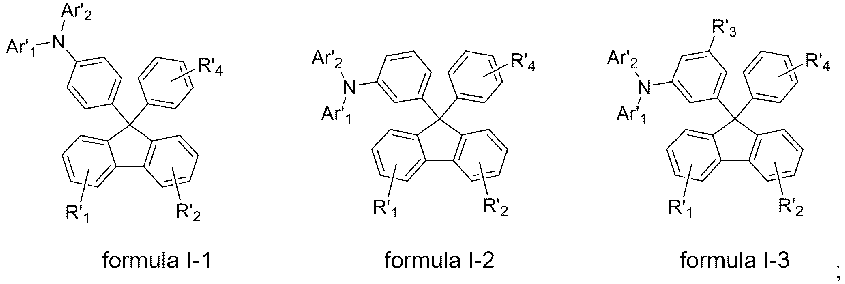 wherein
R'₁-R'₂ are each independently hydrogen, deuterium, methyl, ethyl, isopropyl, n-propyl, tert-butyl, phenyl, naphthyl, biphenyl, pyridinyl, methoxy, phenanthryl, dibenzofuranyl, or dimethylfluorenyl;
R'₃ is deuterium, methyl, ethyl, n-propyl, isopropyl, tert-butyl, phenyl, naphthyl, biphenyl, pyridinyl, methoxy, phenanthryl, dimethylfluorenyl, or furanyl;
Ar'₁ and Ar'₂ are linked to N at any substitutable position;
Ar'₁ and Ar'₂ are each independently one of the following groups or a combination thereof:

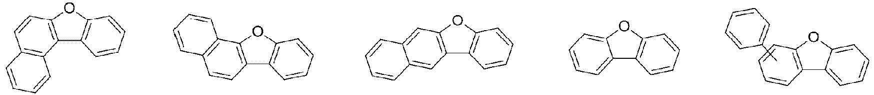

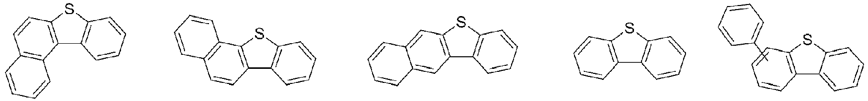

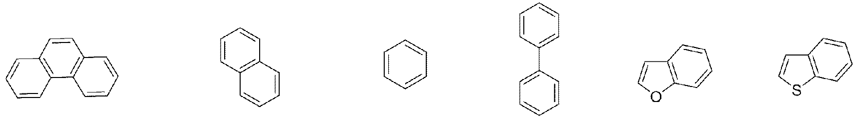

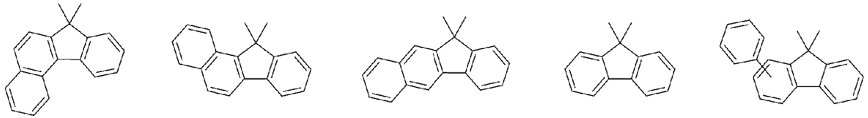

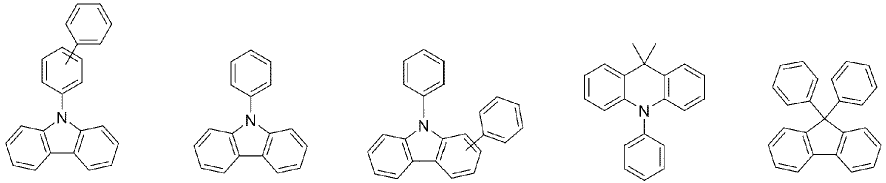

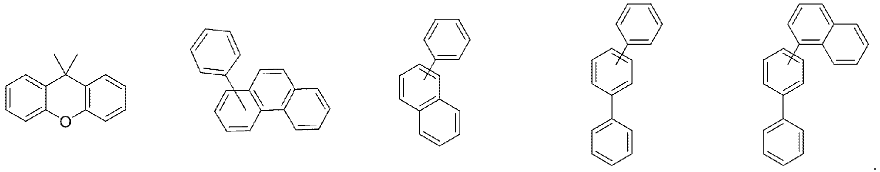

3. The organic electroluminescent device of claim 2, wherein the material of the second hole transport layer is selected from one of the following HT-1 to HT-56:

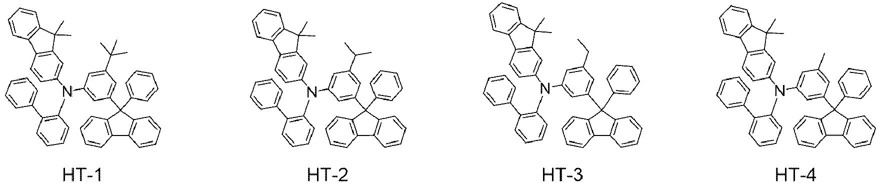

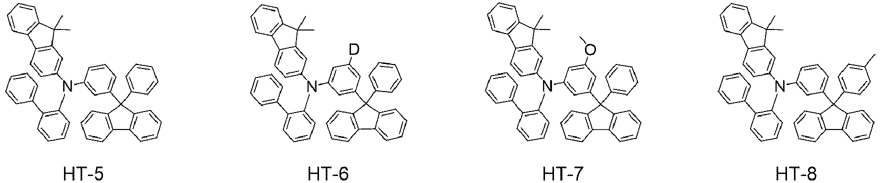

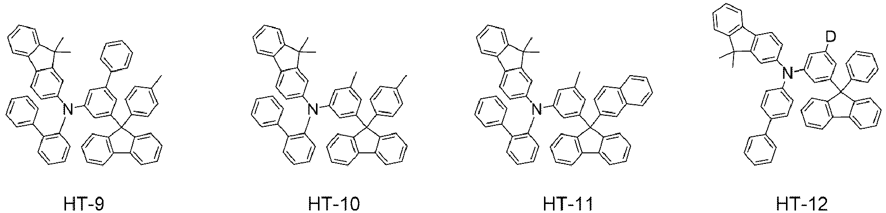

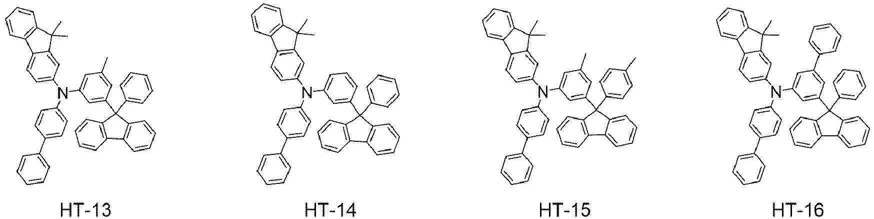

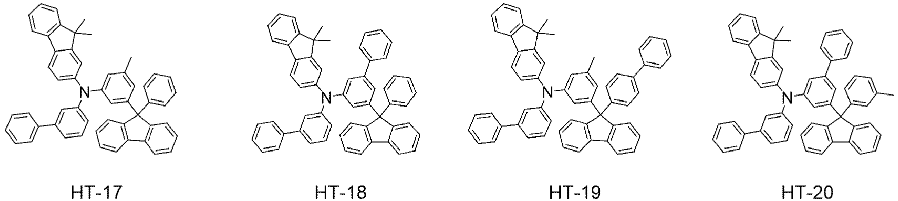

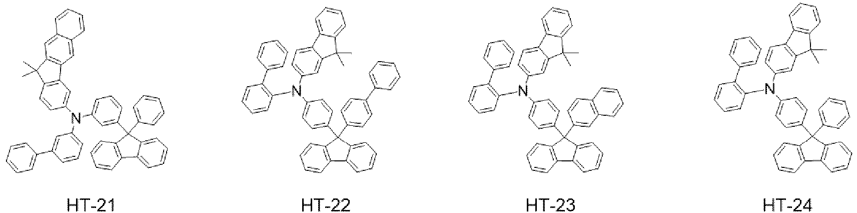

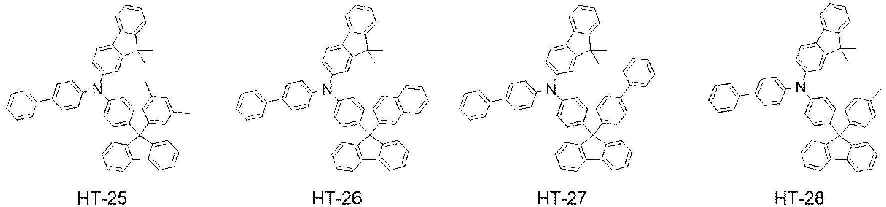

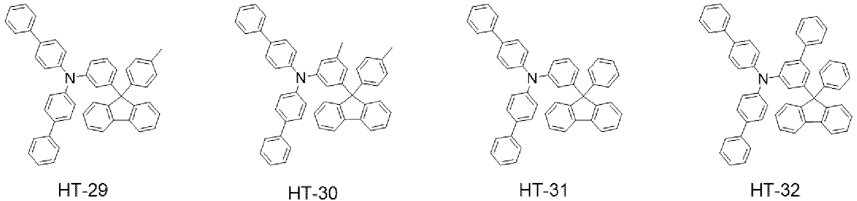

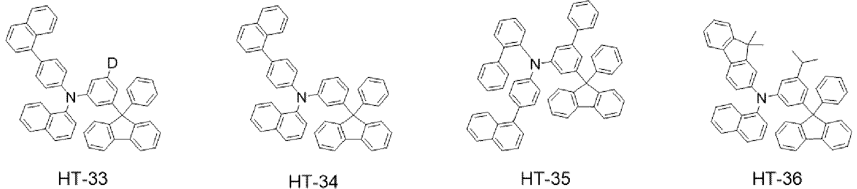

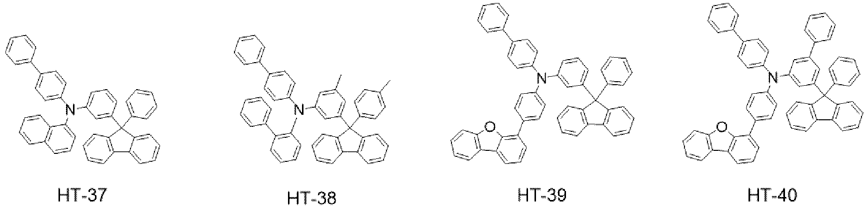

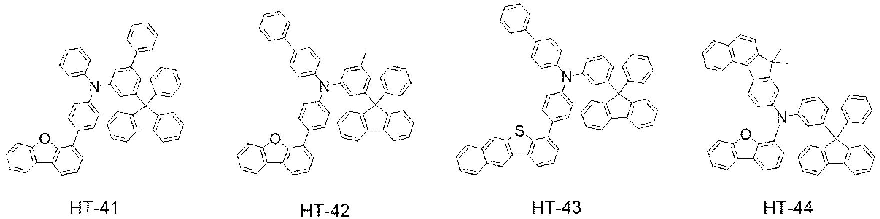

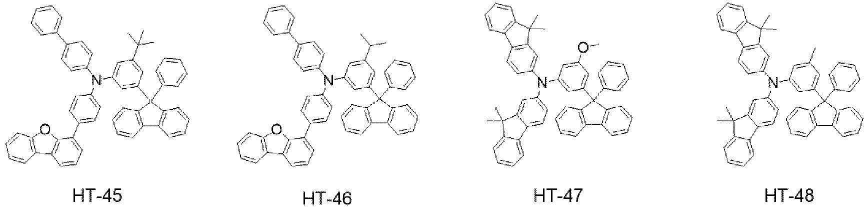

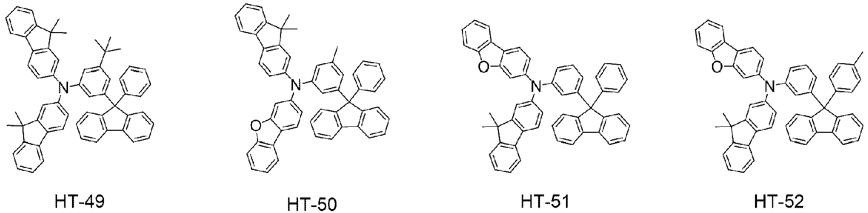

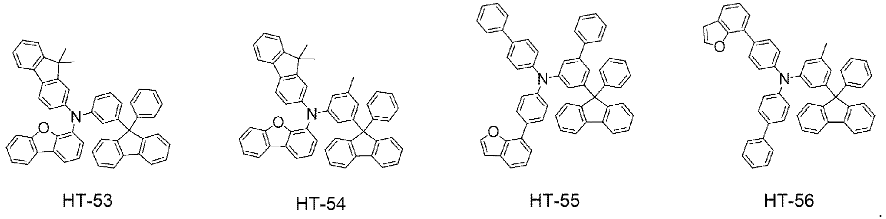

4. The organic electroluminescent device of claim 1, wherein the P-type host material is selected from one of the following formulas II-a to II-d: 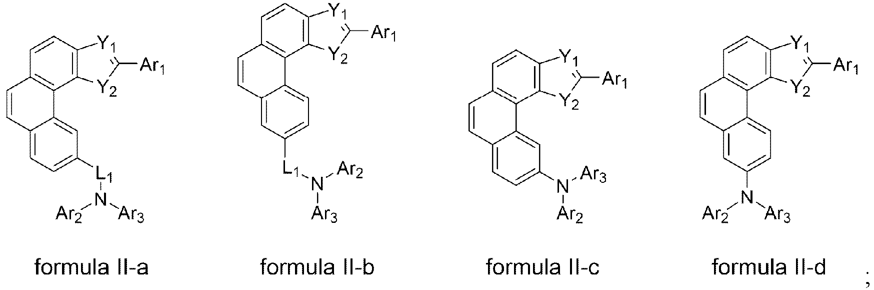 wherein
L₁ is a linking bond, phenyl, naphthyl, biphenyl, terphenyl, pyridinyl, phenanthryl, dibenzofuranyl, dibenzothienyl, or dimethylfluorenyl; and
Ar₁-Ar₃ are each independently selected from phenyl, methylphenyl, ethylphenyl, isopropylphenyl, tert-butylphenyl, naphthyl, biphenyl, terphenyl, pyridinyl, phenanthryl, dibenzofuranyl, dibenzothienyl, dimethylfluorenyl, or carbazolyl.

5. The organic electroluminescent device of claim 4, wherein the P-type host material is selected from one of the following formulae RH1-1 to RH1-85:

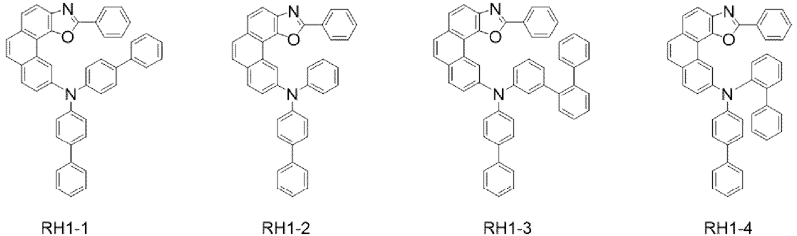

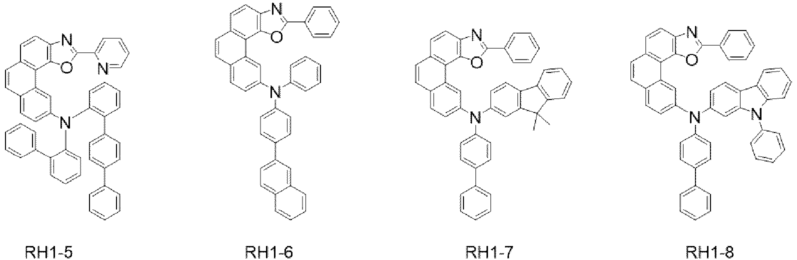

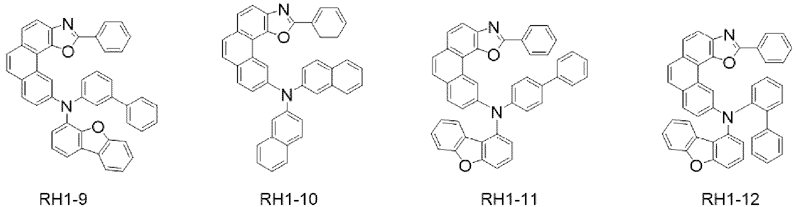

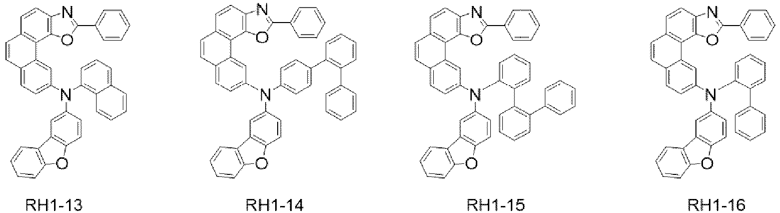

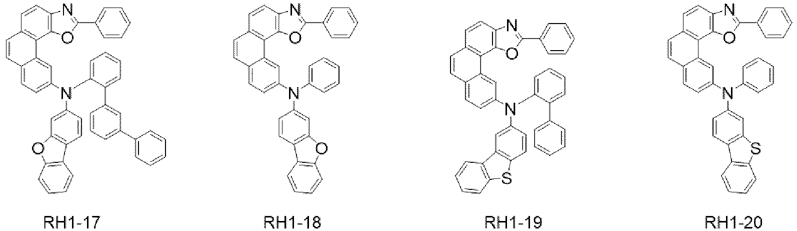

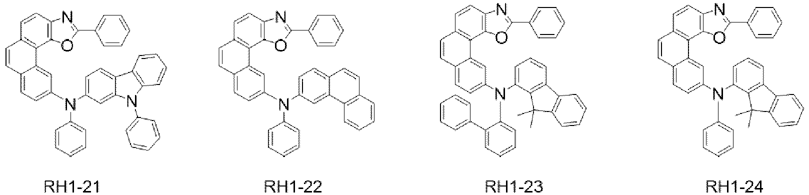

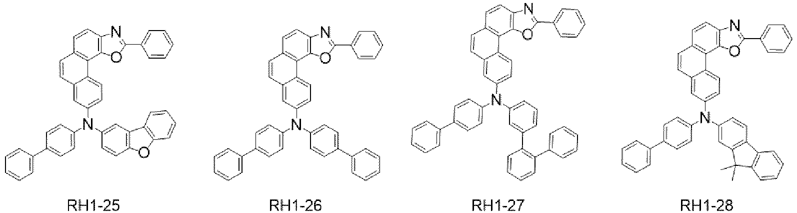

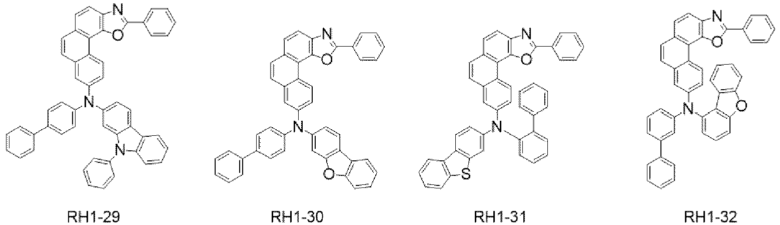

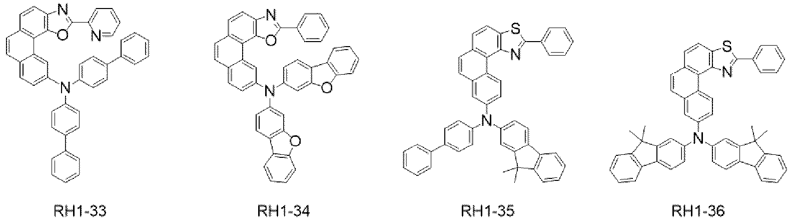

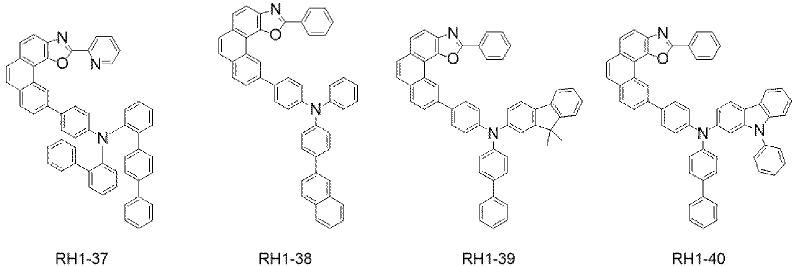

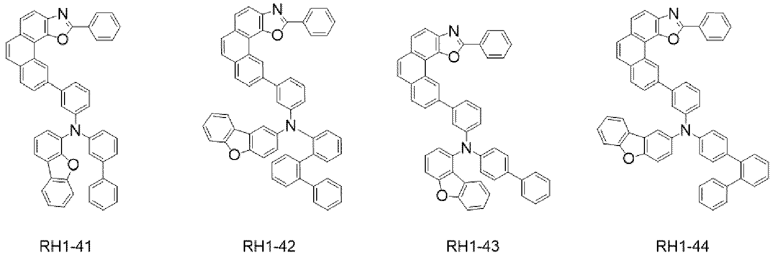

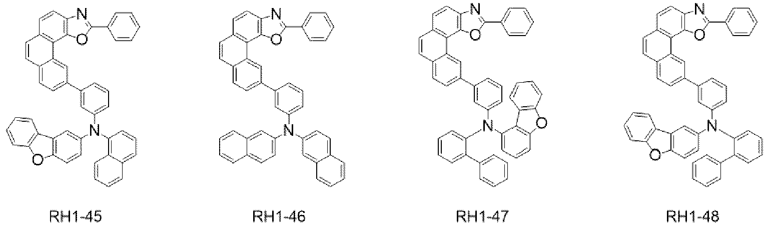

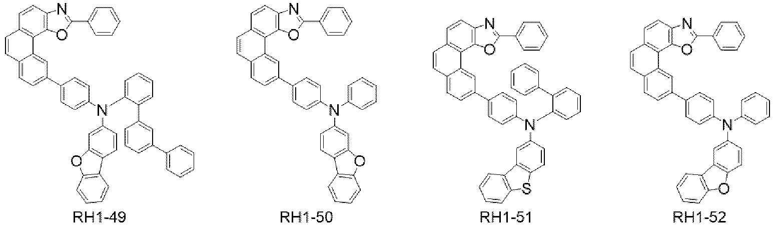

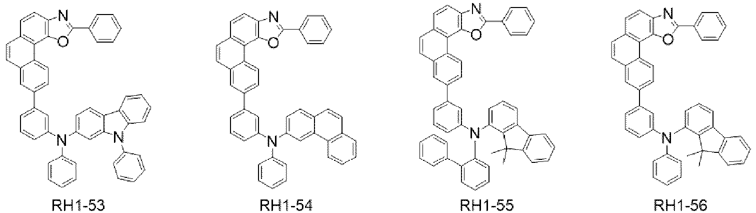

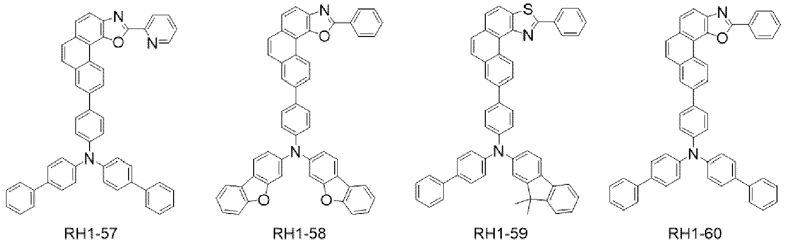

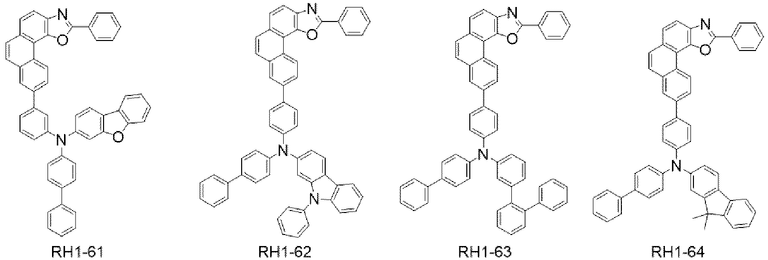

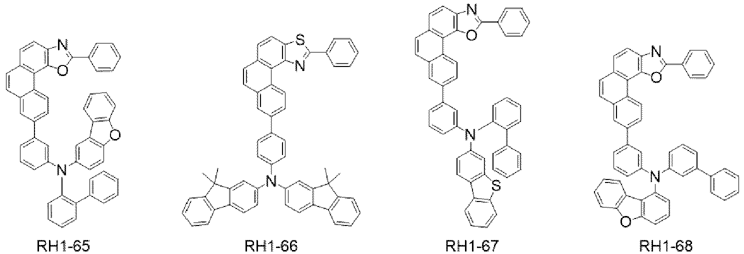

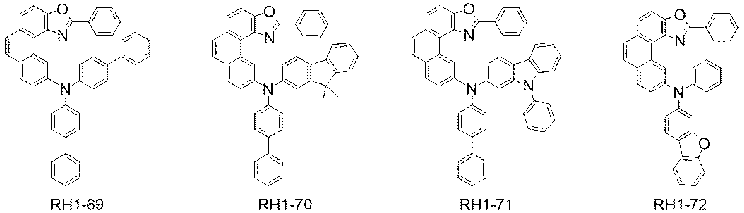

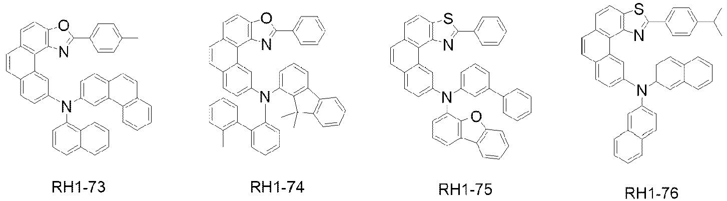

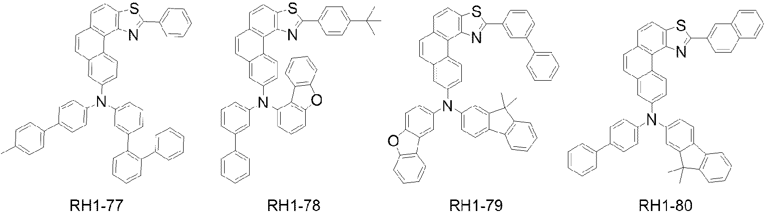

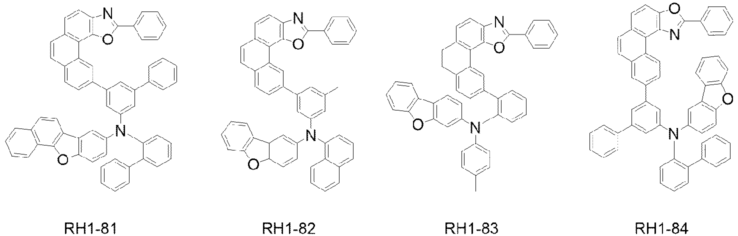

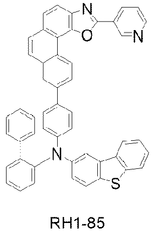

6. The organic electroluminescent device of claim 1, wherein the N-type host material is selected from one of the following formulas III-a to III-c: 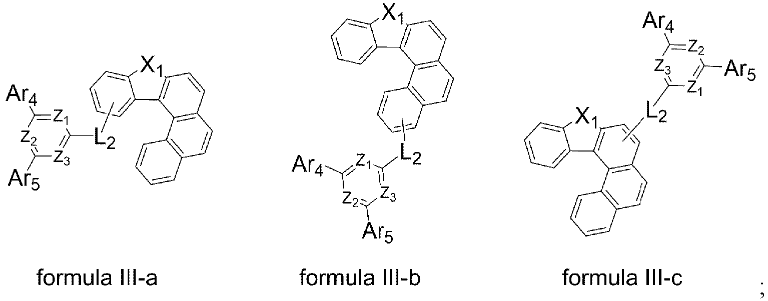 wherein
L₂ is a linking bond, phenyl, naphthyl, biphenyl, pyridinyl, phenanthryl, dibenzofuranyl, dibenzothienyl, or dimethylfluorenyl; and
Ar₄-Ar₅ are each independently selected from phenyl, methylphenyl, ethylphenyl, isopropylphenyl, tert-butylphenyl, naphthyl, biphenyl, terphenyl, pyridinyl, phenanthryl, dibenzofuranyl, dibenzothienyl, dimethylfluorenyl, or carbazolyl.

7. The organic electroluminescent device of claim 6, wherein the N-type host material is selected from one of the following RH2-1 to RH2-96:

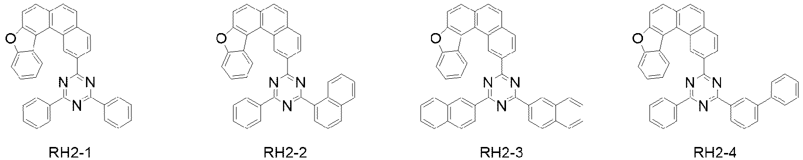

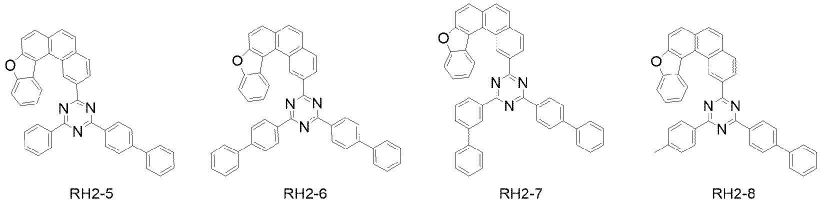

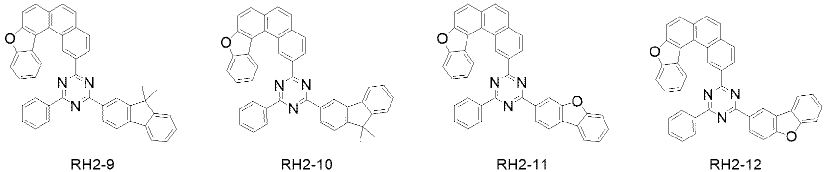

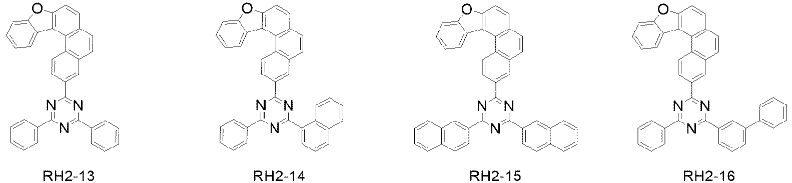

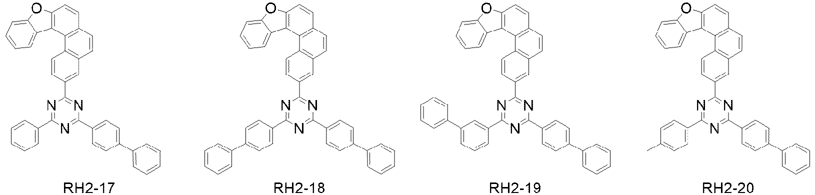

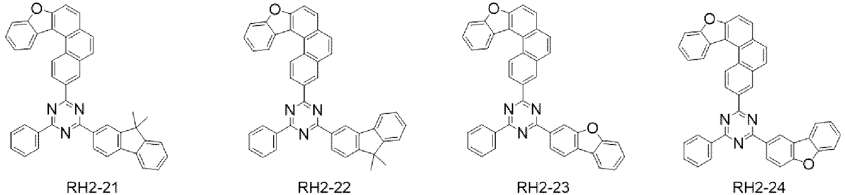

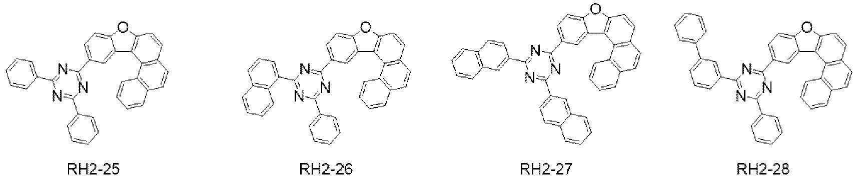

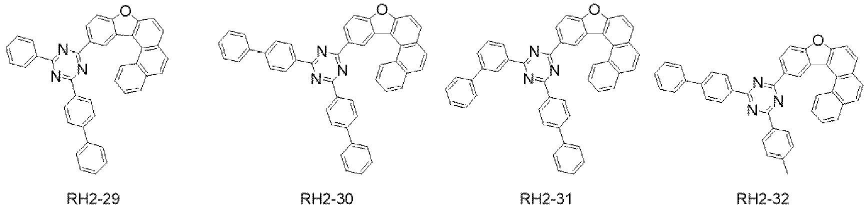

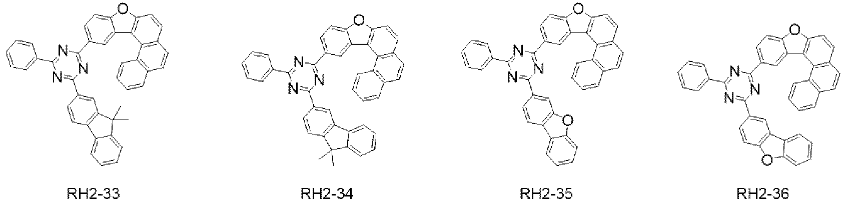

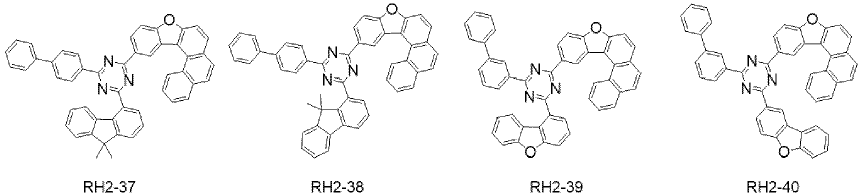

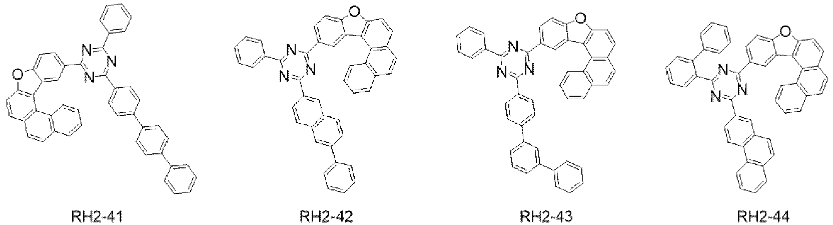

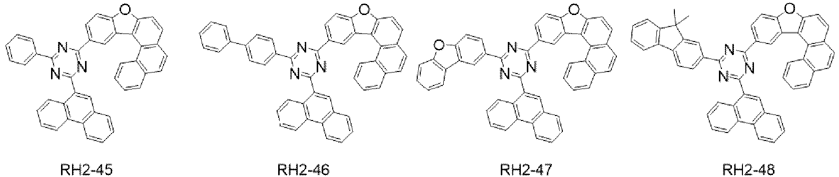

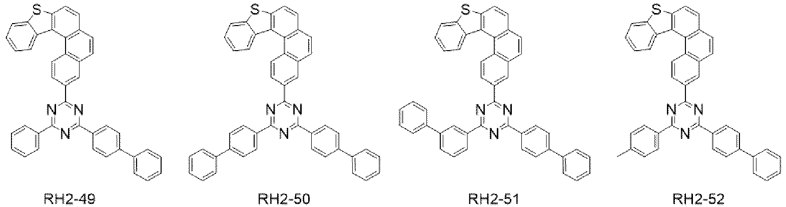

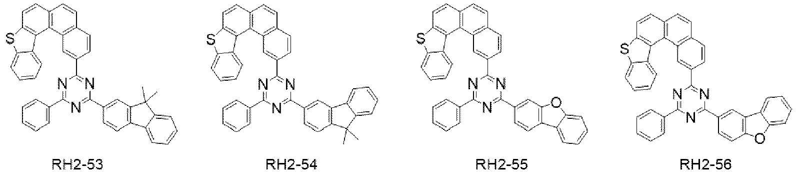

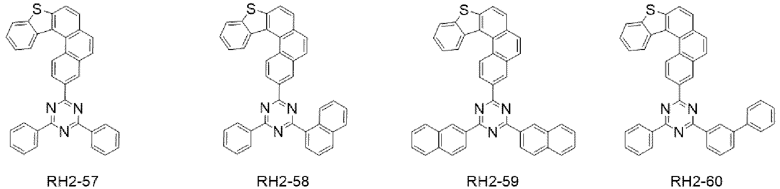

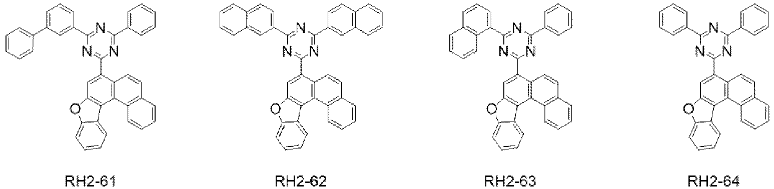

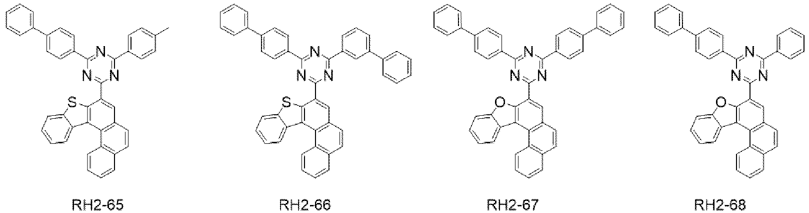

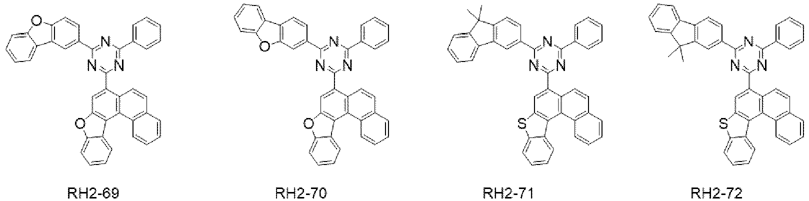

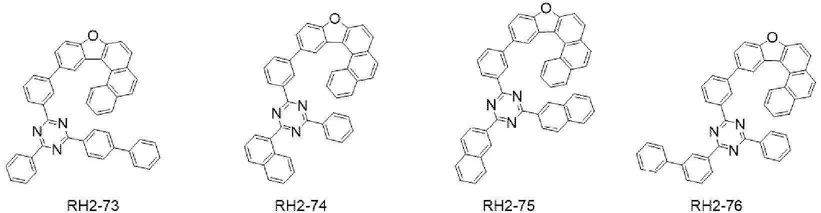

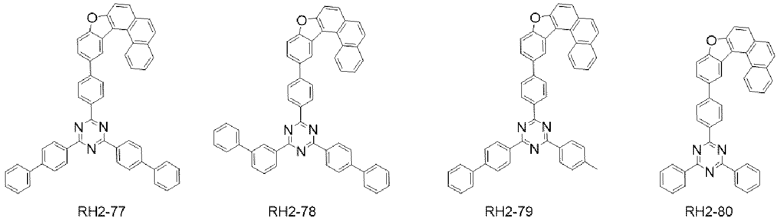

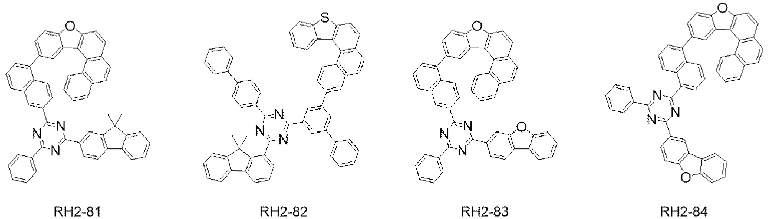

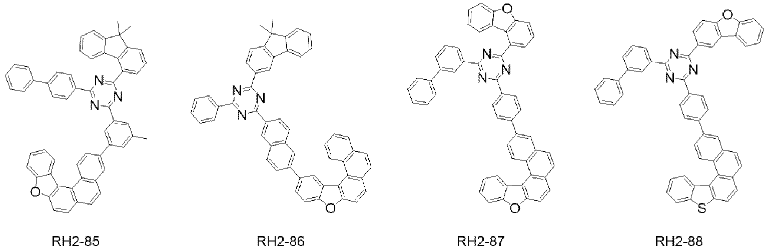

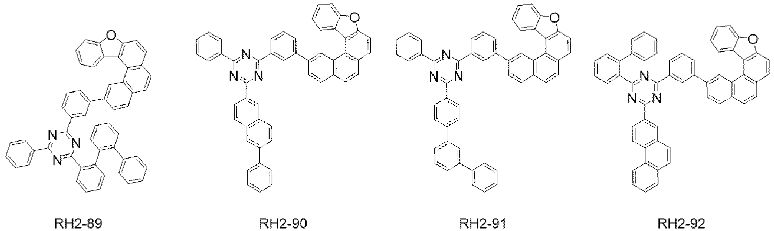

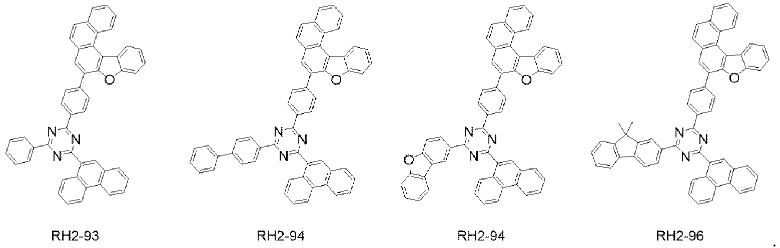

8. An organic electroluminescent apparatus, comprising the organic electroluminescent device of any one of claims 1-7, wherein the organic electroluminescent device is of a top emission type, a bottom emission type, or a dual emission type.

9. A photoelectric device, comprising the organic electroluminescent device of any one of claims 1-7, wherein the photoelectric device is an organic light-emitting device, an organic solar cell, an electronic paper, an organic photoreceptor, or an organic thin-film transistor.
